# EUROPEAN PATENT APPLICATION

(11) **EP 4 410 961 A1**
(43) Date of publication of application: **07.08.2024**
(21) Application number: 22876191.2
(22) Date of filing: 27.09.2022
(51) Int. Cl.: C12N 5/071, A61K 35/30, A61P 5/00, A61P 5/06, A61P 5/10, A61P 5/38, A61P 25/00

(54) **METHOD FOR SEPARATING PITUITARY CELLS AND HYPOTHALAMIC CELLS USING CELL SURFACE MARKER**

(30) Priority: 28.09.2021 JP 2021158406
(71) Applicant: Fujita Academy, Toyoake-shi, Aichi 470-1192 (JP); National University Corporation Tokai National Higher Education and Research System, Nagoya-shi, Aichi 464-8601 (JP)
(72) Inventor: KODANI, Yu, Toyoake-shi, Aichi 470-1192 (JP); KAWATA, Miho, Toyoake-shi, Aichi 470-1192 (JP); NAGASAKI, Hiroshi, Toyoake-shi, Aichi 470-1192 (JP); SUGA, Hidetaka, Nagoya-shi, Aichi 464-8601 (JP)
(74) Representative: J A Kemp LLP
(86) International application number: PCT/JP2022/035857
(87) International publication number: WO 2023/054325

(57) **Abstract**

The present invention provides a method for separating pituitary hormone-producing cells and/or progenitor cells thereof, said method comprising a step for separating CD49c-expressing cells from a cell aggregate including adenohypophysis and/or a progenitor tissue thereof, and a hypothalamic neuroepithelial tissue.

## Description

### [TECHNICAL FIELD]

The present invention relates to a method for separating and purifying pituitary hormone-producing cells and progenitor cells thereof, and hypothalamic cells, induced to differentiate from pluripotent stem cells in vitro.

### [BACKGROUND ART]

The pituitary gland is a small endocrine organ that exists adjacent to the lower part of the diencephalon and plays a major role in the regulation of various hormones. For example, it produces various pituitary hormones including adrenocorticotropic hormone (ACTH), which promotes the production of adrenocortical hormones essential for life support, growth hormone, which promotes the growth of children, and the like. Therefore, pituitary dysfunction causes serious systemic diseases.

In recent years, a method for inducing differentiation of human ES/iPS cells into the adenohypophysis comprising functional pituitary hormone-producing cells has been developed, and its application to hypophyseal regenerative medicine is expected (Patent Literature 1, Non Patent Literatures 1 and 2). In these methods, a hypothalamic-pituitary composite tissue is formed in a cell aggregate by three-dimensional culture. In the composite tissue, the adenohypophysis and its progenitor tissue are mainly located on the surface layer of the cell aggregate. By detaching and transplanting them to under the renal capsule of hypophysectomized mice, therapeutic effects such as improvement of activity, survival, body weight decrease and the like have been confirmed (Patent Literature 1, Non Patent Literature 1). However, in the above-mentioned method, the discrimination of adenohypophysis and progenitor tissue thereof to be detached relies on the subjective judgment made by experimenters based on morphology.

### [CITATION LIST]

### [PATENT LITERATURE]

[PTL 1] WO 2016/013669

### [NON PATENT LITERATURE]

[NPL 1] Nature Communications, 7:10351, 2016
[NPL 2] Cell Reports, 30, 18-24, January 7, 2020

### [SUMMARY OF INVENTION]

### [TECHNICAL PROBLEM]

The present inventors previously identified EpCAM as an objective and reliable cell surface antigen for separating pituitary hormone-producing cells and progenitor cells thereof. It was found that pituitary hormone-producing cells and progenitor cells thereof can be effectively separated and purified from a cell aggregate including adenohypophysis and/or a progenitor tissue thereof by cell sorting using EpCAM as an index. In addition, it was found that the separated and purified cells can be reaggregated and subjected to maintenance culture, that even after the maintenance culture, the cells exhibit superior pituitary hormone secretion ability by physiological stimulation of pituitary hormone secretion, and that the cells exhibit functions equivalent to those of the pituitary hormone-producing cells in the living body. Therefore, the present invention aims to identify a cell surface antigen other than EpCAM is able to separate and purify pituitary hormone-producing cells and/or hypothalamic cells from the cell aggregates, and provide a method for separating a cell of interest by utilizing the cell surface antigen as an index.

### [SOLUTION TO PROBLEM]

The present inventors performed expression analysis of cell surface antigens relating to a group of cells constituting a hypothalamic-pituitary composite tissue, and successfully identified CD49c (integrin α3) as a cell surface antigen highly specific to pituitary cells and hypothalamic cells. As a result of intensive studies, it was found that pituitary cells and hypothalamic cells can be simultaneously separate from cell aggregates derived from pluripotent stem cells by cell sorting the identified cell surface antigen of CD49c as an index, and a cultured tissue having hormone secretion ability corresponding to the hypothalamic-pituitary system can be prepared by reaggregating the separated cells. In addition, it was found that pituitary cells and hypothalamic cells can be separated from each other by cell sorting combining the pituitary cell surface antigen (EpCAM) and CD49c, which resulted in the completion of the present invention.

[1] A method for separating pituitary hormone-producing cells and/or progenitor cells thereof, and hypothalamic cells, comprising a step of separating cells that express CD49c from a cell aggregate comprising adenohypophysis and/or a progenitor tissue thereof, and a hypothalamic neuroepithelial tissue.
[2] A method for separating pituitary hormone-producing cells and/or progenitor cells thereof, comprising a step of separating cells that express CD49c and EpCAM from a cell aggregate comprising adenohypophysis and/or a precursor tissue thereof.
[3] The method of [1] or [2], wherein the step of separating the cells that express CD49c, or the cells that express CD49c and EpCAM comprises the following steps:
   (1) a first step of dispersing the cell aggregate into a cell population,
   (2) a second step of culturing the cell population obtained in the first step on a culture vessel coated with laminin or a fragment thereof, and
   (3) a third step of separating cells that express CD49c or cells that express CD49c and EpCAM from the cell population obtained in the second step.
[4] The method of any of [1] to [3], comprising a step of separating cells negative for at least one cell surface antigen selected from the group consisting of CD90, CD29, CD44, CD58 and CD166.
[5] The method of any of [1] to [4], wherein the cell aggregate is a cell aggregate obtained by inducing differentiation of pluripotent stem cells.
[6] The method of any of [1] to [5], wherein the progenitor tissue is pituitary placode and/or Rathke's pouch.
[7] A method for producing pituitary hormone-producing cells and/or progenitor cells thereof, and hypothalamic cells, comprising a step of separating cells that express CD49c from a cell aggregate comprising adenohypophysis and/or a progenitor tissue thereof, and a hypothalamic neuroepithelial tissue.
[8] A method for producing pituitary hormone-producing cells and/or progenitor cells thereof, comprising a step of separating cells that express CD49c and EpCAM from a cell aggregate comprising adenohypophysis and/or a precursor tissue thereof.
[9] A method for producing a cell aggregate of a cell sheet comprising pituitary hormone-producing cells and/or progenitor cells thereof, and hypothalamic cells, comprising the following steps:
   (A) a step of separating cells that express CD49c from a cell aggregate comprising adenohypophysis and/or a progenitor tissue thereof, and a hypothalamic neuroepithelial tissue,
   (B) a step of reaggregating a population obtained in the step of (A).
[10] A method for producing a cell aggregate or a cell sheet comprising pituitary hormone-producing cells and/or progenitor cells thereof, comprising the following steps:
   (a) a step of separating cells that express CD49c from a cell aggregate comprising adenohypophysis and/or a progenitor tissue thereof,
   (b) a step of reaggregating a population obtained in the step of (a).
[11] The method of any of [7] to [10], wherein the step of separating the cells that express CD49c, or the cells that express CD49c and EpCAM comprises the following steps:
   (1) a first step of dispersing the cell aggregate into a cell population,
   (2) a second step of culturing the cell population obtained in the first step on a culture vessel coated with laminin or a fragment thereof, and
   (3) a third step of separating cells that express CD49c or cells that express CD49c and EpCAM from the cell population obtained in the second step.
[12] The method of any of [7] to [11], comprising a step of separating cells negative for at least one cell surface antigen selected from the group consisting of CD90, CD29, CD44, CD58 and CD 166.
[13] The method of any of [7] to [12], wherein the pituitary hormone-producing cell is at least one type selected from the group consisting of growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, and luteinizing hormone (LH)-producing cells.
[14] A cell aggregate or a cell sheet produced by the method of any of [9] to [13].
[15] A transplant therapeutic agent comprising a cell aggregate or a cell sheet produced by the method of any of [9] to [13].
[16] The transplant therapeutic agent of [15] for treating a disease based on a disorder of adenohypophysis and/or a damaged state of adenohypophysis.
[17] The transplant therapeutic agent of [16], wherein the disease based on the disorder of adenohypophysis is selected from the group consisting of panhypopituitarism, pituitary dwarfism, adrenocortical insufficiency, partial hypopituitarism and pituitary anterior hormone isolated deficiency.
[18] A therapeutic agent for a disease based on a disorder of adenohypophysis or a damaged state of adenohypophysis comprising a cell aggregate or a cell sheet produced by the method of any of [9] to [13].
[19] A method for treating a disease based on a disorder of adenohypophysis or a damaged state of adenohypophysis, comprising transplanting an effective amount of a cell aggregate or a cell sheet produced by the method of any of [9] to [13] in a subject in need thereof.
[20] A cell aggregate or a cell sheet of any of [9] to [13] for use in treating a disease based on a disorder of adenohypophysis or a damaged state of adenohypophysis.
[21] A therapeutic composition comprising a cell aggregate or the cell sheet produced by the method of any of [9] to [13] as an active ingredient.
[22] Use of a cell aggregate of a cell sheet of any of [9] to [13] for the production of a therapeutic agent for a disorder of adenohypophysis or a damaged state of adenohypophysis.

### [ADVANTAGEOUS EFFECTS OF INVENTION]

According to the present invention, by utilizing CD49c, a cell population containing functional pituitary hormone-producing cells and/or progenitor cells thereof, and hypothalamic cells can be efficiently separated and purified from cell aggregates derived from pluripotent stem cells. The separated and purified cell population exhibits superior pituitary hormone secreting ability by physiological stimulation of pituitary hormone secretion, and can function in cooperation with the hypothalamus cells. Therefore, the diseases relating to pituitary gland can be treated using the cell population.

### [BRIEF DESCRIPTION OF DRAWINGS]

[Fig. 1]

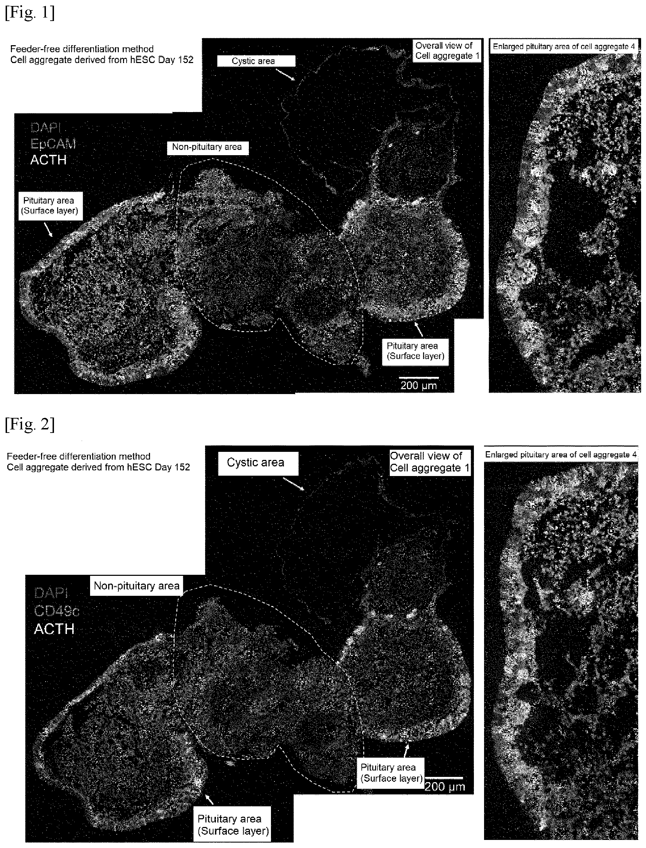

   Fig. 1 shows the results of immunostaining of human ES cell (hESC)-derived cell aggregates (Day 152). EpCAM was confirmed to be expressed in the pituitary area and cystic area.
[Fig. 2]
   Fig. 2 shows the results of immunostaining of hESC-derived cell aggregates (Day 152). CD49c was confirmed to be expressed in the pituitary area and some non-pituitary areas (mainly seems to be hypothalamic nerve tissue).
[Fig. 3]

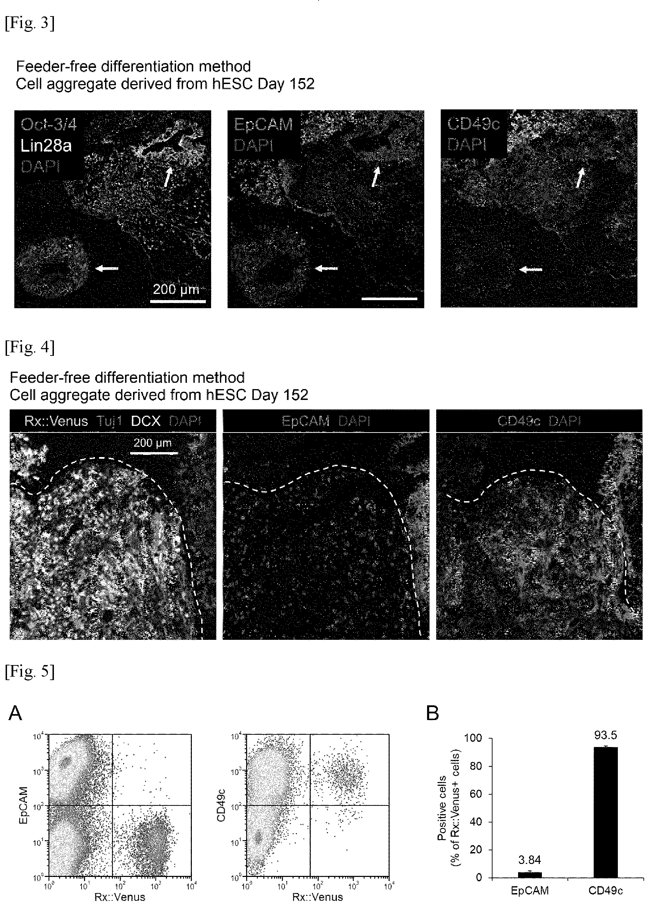

   Fig. 3 shows the results of immunostaining in the undifferentiated area of hESC-derived cell aggregates (Day 152). It was confirmed that EpCAM was expressed in undifferentiated cell areas, but CD49c was not expressed.
[Fig. 4]
   Fig. 4 shows the results of immunostaining on serial sections of undifferentiated areas of hESC-derived cell aggregates (Day 152). The area surrounded by the dotted line is considered to be hypothalamic cells area because it expresses a hypothalamic marker (Rx::Venus) and/or a neuron marker (Tuj 1, DCX). In this hypothalamic cells area, it was confirmed that EpCAM was not expressed, but CD49c was widely expressed.
[Fig. 5]
   A: The left figure shows the results of analyzing the expression of EpCAM and CD49c in Rx::Venus⁺ hypothalamic cells using flow cytometry. B: The right figure shows the results of quantifying the positive rate of EpCAM and CD49c in Rx::Venus⁺ hypothalamic cells with n = 3 based on the analysis results in the left figure A. It was confirmed that CD49c was expressed in Rx::Venus⁺ cells, but EpCAM was hardly expressed.
[Fig. 6]

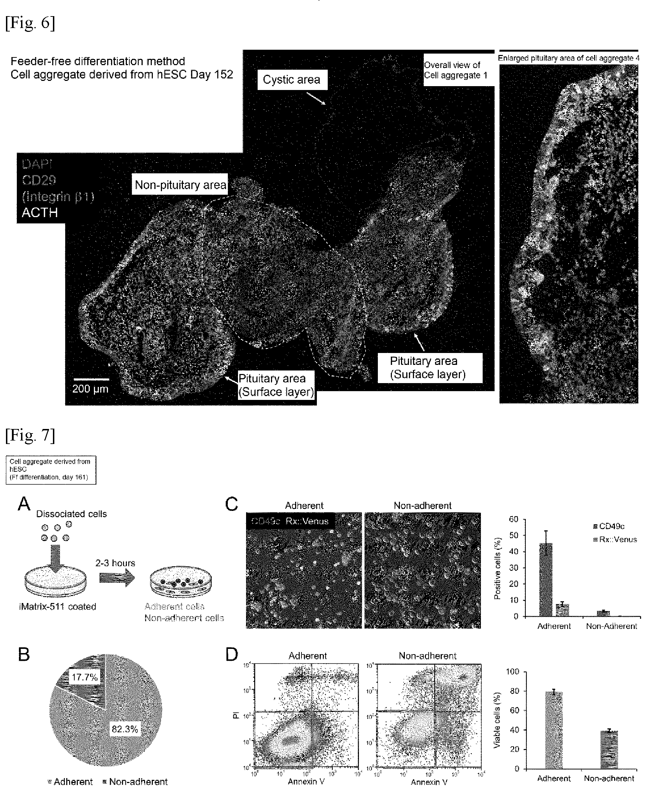

   Fig. 6 shows the results of immunostaining of hESC-derived cell aggregates (Day 152). CD29 (integrin β1) was confirmed to be expressed throughout the tissue except for cystic areas. In comparison with Fig. 2, it is seemed that it forms a dimer with CD49c (integrin α3) (integrin α3/β1).
[Fig. 7]
   Fig. 7 shows a method of cell separation using hESC-derived cell aggregates (Day 161) utilizing the cell adhesive properties of a recombinant active fragment of Laminin 511 (product name: iMatrix-511), which is an extracellular matrix (also referred to as "iMatrix-511 adhesion treatment"). A: The upper left figure shows that cells dispersed by enzymatic treatment of cell aggregates are cultured for 2 to 3 hours in a culture vessel coated with iMatrix-511, and the cells are separated into cells present in the floating cell fraction and cells present in the adherent cell fraction. B: The lower left figure shows the ratio of cells present in the floating cell fraction and cells present in the adhesive cell fraction. C: The upper right figure shows the results of immunostaining of cells present in the floating cell fraction and cells present in the adhesive cell fraction. The right graph of the figure shows the ratio of CD49c-positive cells and Rx:: Venus-positive cells in the floating cell fraction and adhesive cell fraction. It was confirmed that CD49c-positive cells and Rx::Venus-positive cells were collected as an iMatrix-511 adhesive cell fraction. D: The lower right figure shows cell viability in the floating cell fraction and the adhesive cell fraction. It was confirmed that the iMatrix-511 adhesive cell fraction had a higher survival rate than the floating cell fraction. Dead cells can be easily removed by iMatrix-511 adhesion treatment, and only cells with a high survival rate can be subjected to sorting using CD49c as an index.
[Fig. 8]

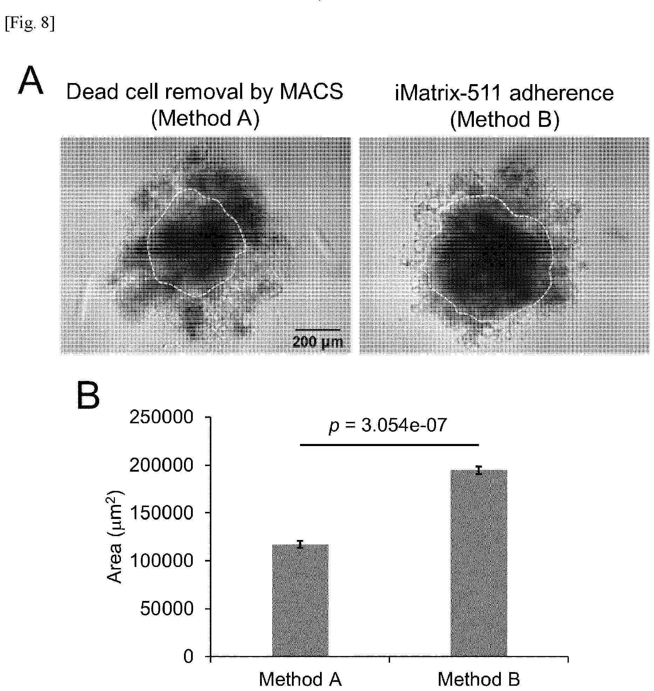

   Fig. 8 shows the improvement in cell survival rate by iMatrix-511 adhesion treatment using reaggregation ability as an index. A: In the upper figure, hESC-derived cell aggregates (Day 448) induced by the feeder-free differentiation method were dispersed, then treated with either Method A or Method B below, reaggregated in groups of 15,000 cells, and observed after 3 days. Method A: Harvest live cells with MACS dead cell removal kit. Method B: Collect iMatrix-511 adhesive cell fraction. The dotted line shows the outline of the aggregates, and the cells surrounding the aggregates were mostly dead cells. B: The lower figure is the result of measuring the area of aggregates (n = 6-8). The p-value is shown in the case of comparing the mean values using the Welch t test. Compared to the conventional sorting pretreatment method (Method A), iMatrix-511 adhesive cell fraction (Method B) was confirmed to have fewer dead cells and form larger aggregates.
[Fig. 9]

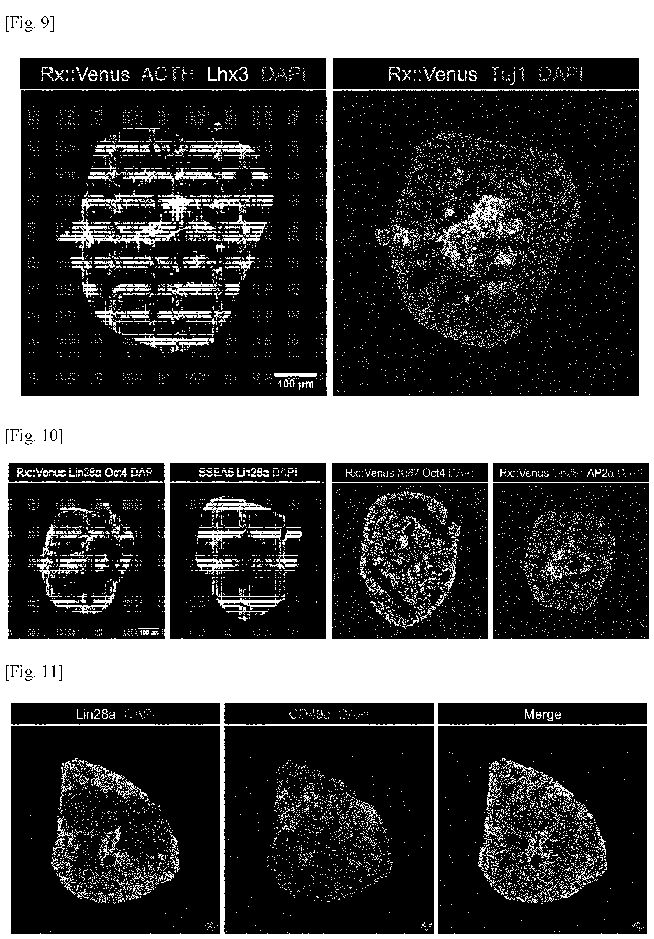

   Fig. 9 shows the results of immunostaining on reaggregates (Day 7) after iMatrix-511 adhesion treatment. In the reaggregates, it was confirmed that pituitary markers (ACTH, Lhx3) and hypothalamic markers (Rx::Venus) were expressed, and many Rx::Venus-positive cells co-express a neuronal marker (Tuj 1). This result demonstrated that pituitary cells and hypothalamic cells were collected as an iMatrix-511adhesive cell fraction.
[Fig. 10]
   Fig. 10 shows the results of immunostaining on the reaggregates (Day 7) after iMatrix-511 adhesion treatment. In the reaggregates, it was confirmed that the outside of the cell aggregates was surrounded by proliferative undifferentiated cells (Lin28a⁺/Oct4⁺/SSEA5⁺/Ki67⁺). Although Lin28a and AP2α were markers for cyst-forming cells (and surface ectoderm), it was confirmed that all Lin28a-positive cells were Oct4-positive and SSEA5-positive undifferentiated cells, and there were no AP2a-positive cells. The above results demonstrated that undifferentiated cells were collected as an iMatrix-511adhesive cell fraction, but cyst-forming cells were not collected.
[Fig. 11]
   Fig. 11 shows the results of immunostaining on reaggregates (Day 7) after iMatrix-511 adhesion treatment. It was confirmed that CD49c was not expressed in Lin28a-positive areas (undifferentiated cells), but that CD49c was expressed in Lin28a-negative areas (including pituitary cells and hypothalamic cells). From this result, it was considered that pituitary cells and hypothalamic cells were able to be purified by separating CD49c-positive cells from the iMatrix-511 adhesive cell fraction.
[Fig. 12]

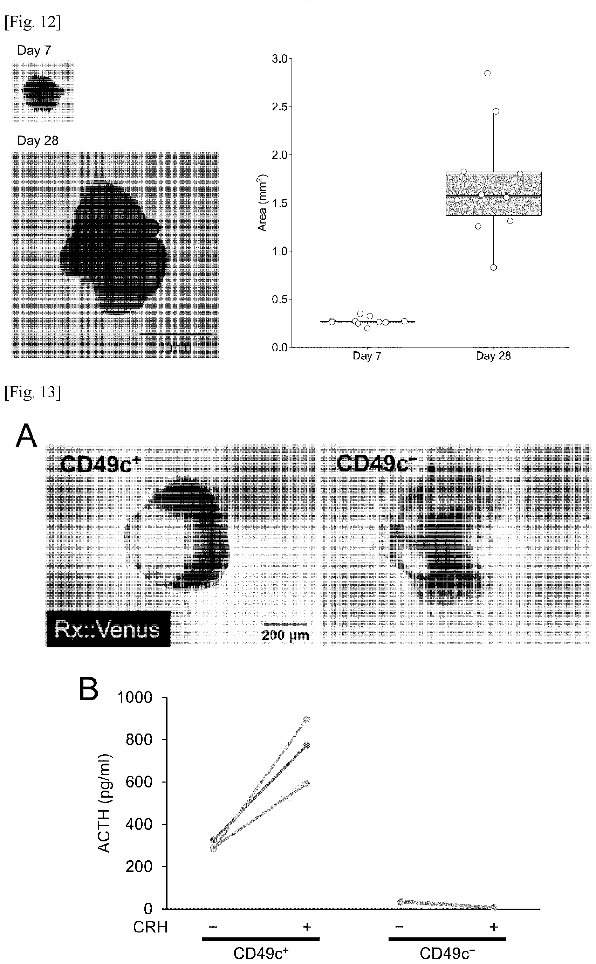

   Fig. 12 shows a comparison of the size of reaggregates on day 7 and day 28 after iMatrix-511 adhesion treatment. As shown in Fig. 10, since the outside of the cell aggregates are surrounded by proliferative undifferentiated cells (Lin28a⁺/Oct4⁺/SSEA5⁺/Ki67⁺), it seems that the reaggregates become enlarged due to the proliferation of undifferentiated cells.
[Fig. 13]
   Fig. 13 shows ACTH secretion ability after separating CD49c-positive cells and CD49c-negative cells by magnetic cell separation (MACS). The test method used cell aggregates that were cultured for 8 days after MACS purification from a sample differentiated from hESC (also referred to as "hESC sample") and reaggregation. 5 cell aggregates were transferred to a 1.5 ml microtube and incubated in 250 µl of HBSS(+) (Wako, # 084-08965) at 37°C for 10 minutes, and then HBSS was collected. Subsequently, HBSS was collected after incubation at 37°C for 10 minutes in 250 µl of HBSS containing 1 µg/ml CRH (Peptide Institute, #4136-s). The ACTH concentration in the collected HBSS was measured by the ECLIA method used in clinical testing. A: The upper figure shows an image taken 7 days after reaggregation of CD49c-positive cells and CD49c-negative cells separated by MACS. B: The lower figure shows the results of measuring the amount of ACTH secretion before and after CRH stimulation. It was confirmed that CD49c-positive cells had a high basal secretion of ACTH and increased ACTH secretion in response to CRH, a physiological secretion stimulating factor, whereas CD49c-negative cells had a low basal secretion of ACTH and did not respond to CRH. This demonstrated that ACTH cells were separated into CD49c-positive cells.
[Fig. 14]

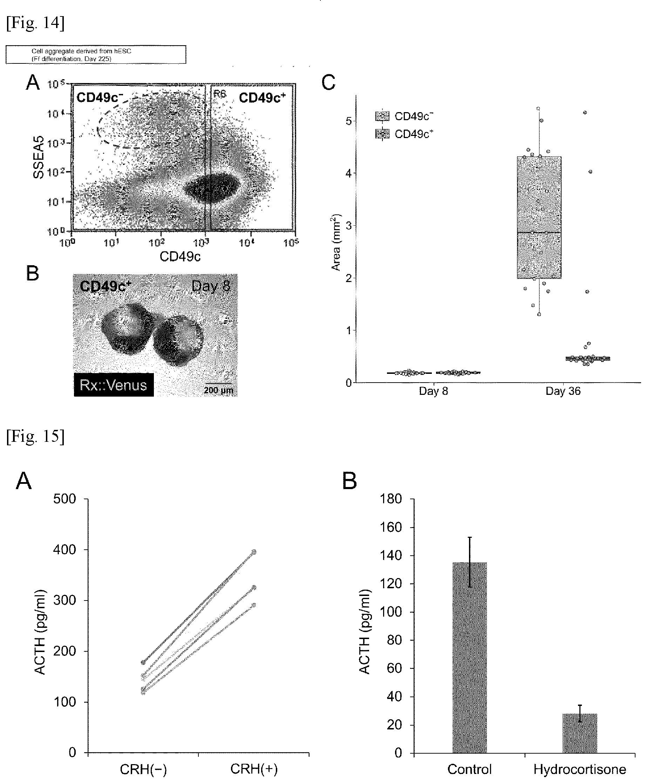

   Fig 14. shows the results of determining the fluorescence intensity range of CD49c-positive cells and CD49c-negative cells by fluorescence-activated cell sorting (FACS), separating the CD49c-positive cells and CD49c-negative cells and comparing the size of the reaggregates on day 8 and Day 36. A: The upper left figure shows an example of FACS. Due to the autofluorescence of the cells, it is difficult to distinguish between CD49c-positive cells and CD49c-negative cells using CD49c fluorescence staining alone. Therefore, simultaneous fluorescent staining of CD49c and SSEA5, a marker of undifferentiated cells, is used as an index for determining the fluorescence intensity range of CD49c-negative cells. Cell populations that highly express SSEA5 (dotted line area) are undifferentiated cells. In subsequent experiments, cell populations obtained by sorting CD49c-positive cells were used to avoid contamination with undifferentiated cells. B: The lower left figure shows the reaggregates of separated CD49c-positive cells on day 8 (Day 8). C: The right figure shows the results of quantifying the size (area) of the reaggregates. It was confirmed that in CD49c-positive cells, the enlargement of cell aggregates was significantly suppressed by removing proliferative cells including undifferentiated cells. Furthermore, it can be seen that on Day 36 of CD49c-positive cells, there are cell aggregates with a large change in area. Since these are considered to be cell aggregates in which proliferative cells remain, therapeutic cells can be purified by removing these cell aggregates based on area or morphology.
[Fig. 15]
   Fig 15 shows ACTH secretion ability after separating CD49c-positive cells by FACS and reaggregating the cells. A: The left figure shows the results of measuring the amount of ACTH secretion per cell aggregate before and after CRH stimulation. It was confirmed that ACTH secretion increases in response to CRH, a physiological secretion stimulating factor. As a test method, a cell aggregate that was reaggregated after FACS purification from a hESC sample and cultured for 12 days was used. Each cell aggregate was transferred to a 1.5 ml microtube, incubated in 250 µl of standard glucose medium (glucose-free DMEM + 5.56 mM D-glucose + 4.44 mM D-mannitol) at 37°C for 30 minutes, and then the medium was collected. Subsequently, the aggregate was incubated in 250 µl of standard glucose medium containing 1 µg/ml CRH (Peptide Institute, #4136-s) at 37°C for 30 minutes, and then the medium was collected. The ACTH concentration in the collected medium was measured by the ECLIA method used in clinical testing. B: The right figure shows the results of measuring the amount of ACTH secreted per cell aggregate before and after treatment with cortisol (Hydrocortisone), a physiological secretion suppressor. It was confirmed that ACTH secretion was reduced by cortisol (Hydrocortisone) treatment. It was demonstrated that ACTH cells maintain physiological secretory function in CD49c-positive cell aggregates after sorting by FACS. As a test method, a cell aggregate that was reaggregated after FACS purification from a hESC sample and cultured for 13 days was used. Before secretion measurement, cells were pretreated for 3 hours with maintenance medium (gfCDM + 20%KSR + 2 µM SAG) containing cortisol (Hydrocortisone, 20 µg/ml) or the same amount of solvent (0.1% DMSO) as a control. Each pretreated cell aggregate was transferred to a 1.5 ml microtube, incubated in 250 µl of standard glucose medium (glucose-free DMEM + 5.56 mM D-glucose + 4.44 mM D-mannitol) at 37°C for 30 minutes, and then the medium was collected. Cortisol or DMSO was also continuously added to the above standard glucose medium. The ACTH concentration in the collected medium was measured by the ECLIA method used in clinical testing.
[Fig. 16]
   As a test method, a cell aggregate that was reaggregated after FACS purification from a hESC sample and cultured for 13-15 days was used. Before secretion measurement, cells were pretreated for 3 hours with maintenance medium (gfCDM + 20%KSR + 2 µM SAG) containing antalarmin (10 µM) or the same amount of solvent (0.1% DMSO) as a control. Each pretreated cell aggregate was transferred to a 1.5 ml microtube, incubated in 250 µl of standard glucose medium (glucose-free DMEM + 5.56 mM D-glucose + 4.44 mM D-mannitol) at 37°C for 30 minutes, and the medium was collected. Subsequently, the aggregate was incubated in 250 µl of low glucose medium (glucose-free DMEM + 0.56 mM D-glucose + 9.44 mM D-mannitol) at 37°C for 30 minutes, and then the medium was collected. Antalarmin or DMSO was continuously added to the standard glucose medium and low glucose medium described above. The results of measuring the ACTH concentration in the collected medium using the ECLIA method used in clinical tests are shown. It was confirmed that ACTH secretion increased under low glucose conditions (10 mg/dL) compared to standard glucose conditions (100 mg/dL). From this, it was demonstrated that the regulation was performed in a manner similar to that of a living body. It was also confirmed that treatment with Antalarmin (CRH receptor inhibitor) reduced ACTH secretion under both standard glucose and low glucose conditions. It is seemed that ACTH secretion from pituitary cells is regulated within one cell aggregate via CRH derived from hypothalamic cells. Therefore, it is suggested that the functional connection between hypothalamic cells and pituitary cells can be reestablished in the reaggregate of CD49c-positive cells.
[Fig. 17]

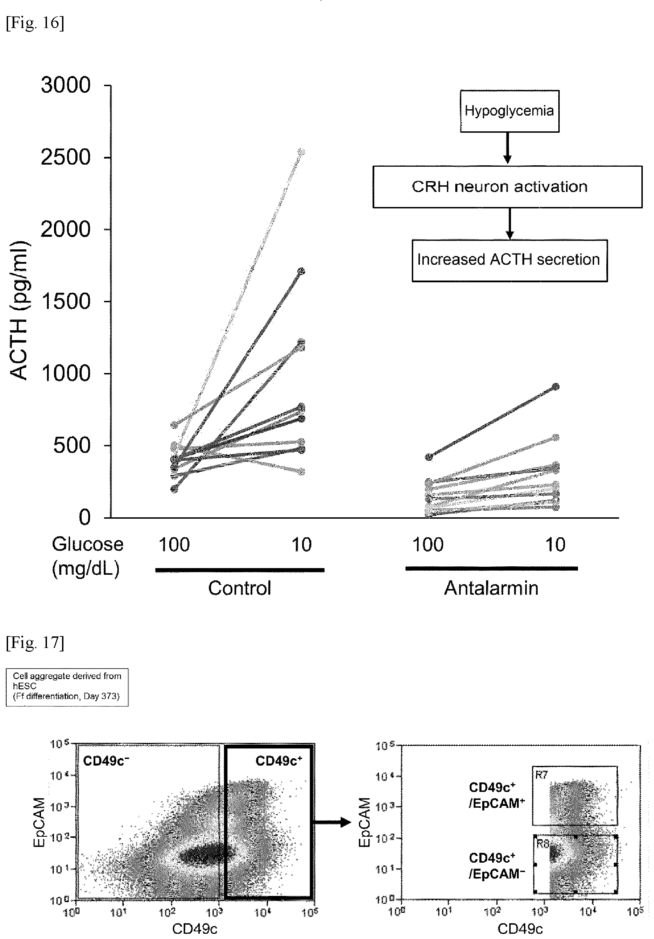

   Fig. 17 shows the fluorescence intensity range of CD49c positive cells/EpCAM positive cells and CD49c positive cells/EpCAM negative cells using FACS. From the results of this study and previous studies by the inventors, it is presumed that pituitary cells can be separated by sorting CD49c-positive cells/EpCAM-positive cells, and hypothalamic cells can be separated by sorting CD49c-positive cells/EpCAM-negative cells.
[Fig. 18]

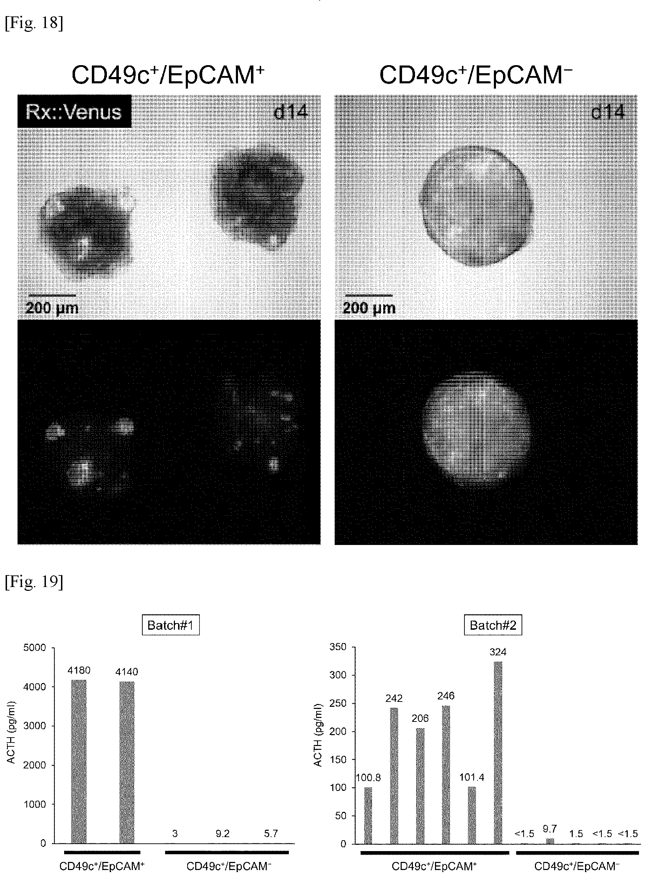

   Fig. 18 shows the expression pattern of Rx:: Venus in reaggregates. It was confirmed that Rx::Venus positive cells (= hypothalamic cells) were concentrated in the CD49c positive/EpCAM negative fraction.
[Fig. 19]
   Fig. 19 shows the ACTH secretion ability of reaggregates after FACS. As a test method, a cell aggregate that was reaggregated after FACS purification from a hESC sample and cultured for 8 days or more was used. After culturing each cell aggregate in 250 µl of maintenance medium (gfCDM + 20%KSR + 2 µM SAG) at 37°C for 24 hours, the medium was collected. The ACTH concentration in the collected medium was measured by the ECLIA method used in clinical testing. ACTH cells were confirmed to be concentrated in the CD49c-positive cell/EpCAM-positive cell fraction.
[Fig. 20]

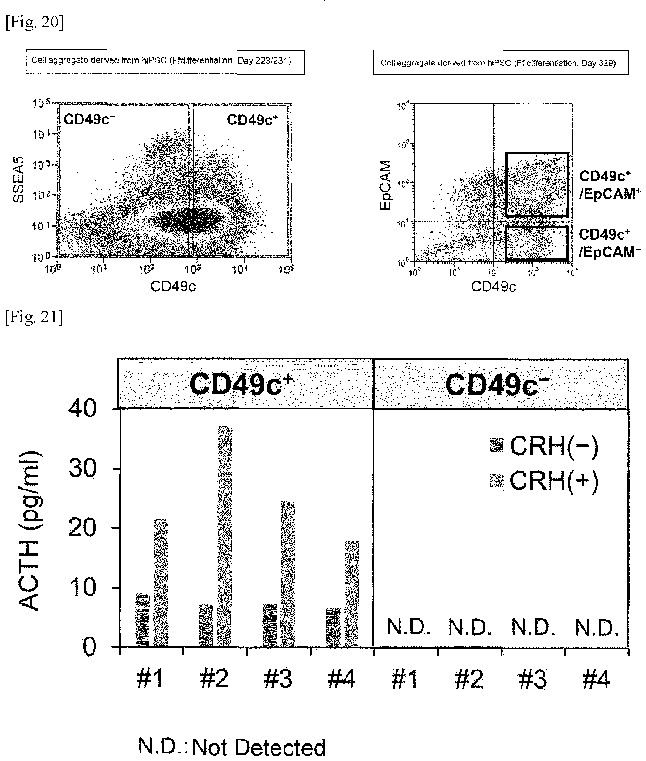

   Fig. 20 shows surface marker expression patterns in samples differentiated from human iPS cells (hiPSCs) (also referred to as "hiPSC sample"). The above all experiments were performed using samples differentiated from hESCs (also referred to as "hESC sample"), and then by comparing with Fig. 14, etc., it was confirmed that the hiPSC sample had a similar expression pattern of surface markers.
[Fig. 21]
   Fig. 21 shows the results of measuring the amount of ACTH secretion per cell aggregate before and after CRH stimulation with hiPSC samples. As a test method, a cell aggregate that was reaggregated after FACS purification from a hiPSC sample and cultured for 21 days was used. Each cell aggregate was transferred to a 1.5 ml microtube, incubated in 250 µl of standard glucose medium (glucose-free DMEM + 5.56 mM D-glucose + 4.44 mM D-mannitol) at 37°C for 30 minutes, and then the medium was collected. Subsequently, the aggregate was incubated in 250 µl of standard glucose medium containing 1 µg/ml CRH (Peptide Institute, #4136-s) at 37°C for 30 minutes, and then the medium was collected. The ACTH concentration in the collected medium was measured by the ECLIA method used in clinical testing. It was confirmed that CD49c-positive cells had a high basal secretion of ACTH and increased ACTH secretion in response to CRH, a physiological secretion stimulating factor, whereas CD49c-negative cells had a low basal secretion of ACTH and did not respond to CRH. This demonstrated that ACTH cells were separated into CD49c-positive cells in hiPSC samples as well as in hESC-derived cells.
[Fig. 22]

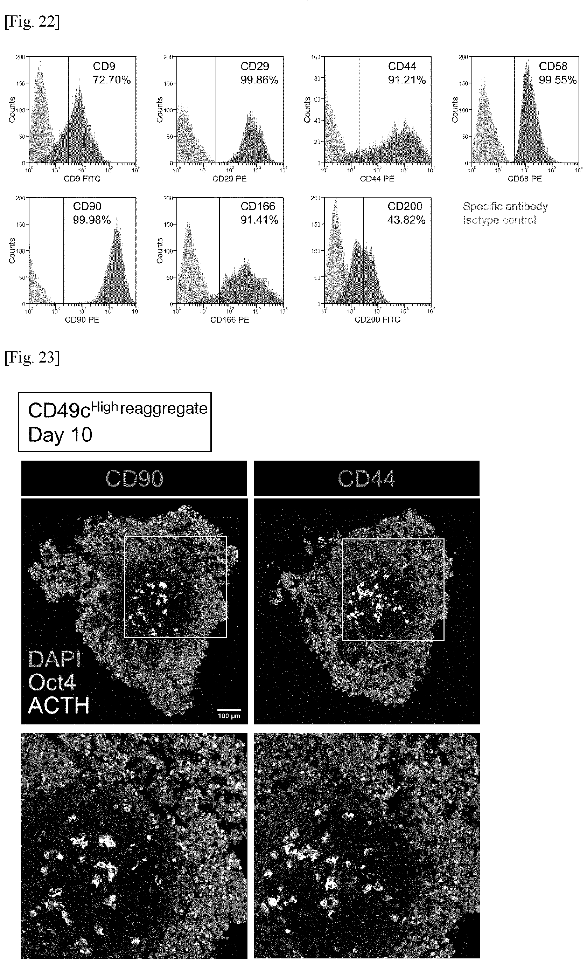

   Fig. 22 shows the results of surface marker search for mesenchymal stem cell-like proliferative cells using a detached tissue. The proliferative cells appear as a detached tissue from cell aggregates in culture. The detached tissue from the hiPSC sample was collected, treated with 10x TrypLE select to disperse (almost completely single cells), fluorescently stained for surface markers of stem cell lineage, and the positive rate was quantified using a flow cytometer. Among the cell surface markers examined, expression of the six types of markers shown in the figure was confirmed in the detached tissue. These are known to be expressed in mesenchymal stem cells (MSCs), and CD90 in particular is one of the major markers highly expressed in MSCs. From these results, it was confirmed that proliferative cells in the detached tissue express surface antigens common to those of mesenchymal stem cells. Particularly, it was confirmed that CD29, CD44, CD58, CD90 and CD166 were expressed in 90% or more of the cells.
[Fig. 23]
   Fig. 23 shows the results of immunostaining on the reaggregates of hiPSC samples. Among the surface antigens shown in Fig. 22, CD90 and CD44 were confirmed to be specifically expressed on mesenchymal stem cell-like proliferative cells (Oct4-positive cells). From this result, it was demonstrated that CD90 and CD44 are preferable as negative markers for removing the proliferative cells.

### [DESCRIPTION OF EMBODIMENTS]

The present invention provides a method for separating pituitary hormone-producing cells and/or progenitor cells thereof, and optionally hypothalamic cells from a cell aggregate including adenohypophysis and/or a progenitor tissue thereof and optionally a hypothalamic neuroepithelial tissue (hereinafter, sometimes referred to as "cell aggregate before cell separation") (hereinafter, sometimes referred to as "separation method of the invention"). The separation method of the invention is characterized by including a step of separating cells from the above-mentioned cell aggregates by expression of CD49c and/or EpCAM as an index. Cell aggregates using the separation method of the invention, typically, can be obtained by culturing cell aggregates of pluripotent stem cells.

### (1) Pluripotent stem cell

The "pluripotent stem cell" refers to a cell that has both the ability to differentiate into all cells constituting the body (differentiation pluripotency) and the ability to produce daughter cells having the same differentiation potency as the self through cell division (self-renewal potential).

The differentiation pluripotency can be evaluated by transplanting the cells to be evaluated into nude mice and examining the presence or absence of teratoma formation containing cells of each of the three germ layers (ectoderm, mesoderm, endoderm).

Examples of the pluripotent stem cell include embryonic stem cells (ES cell), embryonic germ cells (EG cell), induced pluripotent stem cells (iPS cell), embryonal carcinoma cells (EC cell), and the like, and are not limited to these as long as the cell has both the differentiation pluripotency and the self-renewal potential. In the present invention, ES cell or iPS cell (more preferably human iPS cell) is preferably used. When the above-mentioned pluripotent stem cell is any cell derived from ES cell or human embryo, the cell may be a cell produced by destroying an embryo or without destroying an embryo, preferably a cell produced without destroying an embryo. Human ES cells used in the present invention are the ones established from human embryos within 14 days of fertilization.

ES cell can be established by, for example, culturing early embryo before implantation, inner cell mass constituting the early embryo, single blastomere, and the like (Manipulating the Mouse Embryo A Laboratory Manual, Second Edition, Cold Spring Harbor Laboratory Press (1994), Thomson, J. A. et al., Science, 282, 1145-1147 (1998)). As the early embryo, an early embryo produced by nuclear transfer of the nucleus of a somatic cell may also be used (Wilmut et al., (Nature, 385, 810 (1997)), Cibelli et al. (Science, 280, 1256 (1998)), Akira Iritani et al. (protein nucleic acid enzyme, 44, 892 (1999)), Baguisi et al., (Nature Biotechnology, 17, 456 (1999)), Wakayama et al., (Nature, 394, 369 (1998); Nature Genetics, 22, 127 (1999); Proc. Natl. Acad. Sci. USA, 96, 14984 (1999)), Rideout III et al., (Nature Genetics, 24, 109 (2000)), Tachibana et al., (Human Embryonic Stem Cells Derived by Somatic Cell Nuclear Transfer, Cell (2013) in press)). As the early embryo, parthenogenetic embryo may also be used (Kim et al., (Science, 315, 482-486 (2007)), Nakajima et al., (Stem Cells, 25,983-985 (2007)), Kim et al., (Cell Stem Cell, 1, 346-352 (2007)), Revazova et al., (Cloning Stem Cells, 9, 432-44β (2007)), Revazova et al., (Cloning Stem Cells, 10, 11-24 (2008))).

Fused ES cells obtained by cell fusion of ES cells and somatic cells are also included in the ES cells to be used in the methods of the present invention.

ES cells can be obtained from designated institutions, and commercially available products can also be purchased. For example, human ES cells KhES-1, KhES-2, and KhES-3 are available from The Institute for Frontier Life and Medical Sciences, Kyoto University.

EG cells can be established by culturing primordial germ cells in the presence of LIF, bFGF, SCF, and the like (Matsui et al., Cell, 70, 841-847 (1992), Shamblott et al., Proc. Natl. Acad. Sci. USA, 95(23), 13726-13731 (1998), Turnpenny et al., Stem Cells, 21(5), 598-609, (2003)).

iPS cells refer to cells that have artificially acquired differentiation pluripotency and self-renewal potential by contacting somatic cells (e.g., fibroblast, skin cell, lymphocyte, etc.) with nuclear reprogramming factors. iPS cells were first discovered by a method of introducing nuclear reprogramming factors consisting of Oct3/4, Sox2, Klf4, and c-Myc into somatic cells (e.g., fibroblast, skin cell, etc.) (Cell, 126: p. 663-676, 2006). Since then, many researchers have made various improvements to combinations of reprogramming factors and methods of introducing factors, and various production methods of iPS cells have been reported.

The nuclear reprogramming factor may be configured with any substance, such as a proteinous factor or a nucleic acid that encodes the same (including a form integrated in a vector), or a low molecular compound, as long as it is a substance (substances) capable of inducing a cell having differentiation pluripotency and self-renewal potential from somatic cells such as fibroblast and the like. When the nuclear reprogramming factor is a proteinous factor or a nucleic acid that encodes the same, preferable nuclear reprogramming substance is exemplified by the following combinations (hereinafter, only the names for proteinous factors are shown).
(1) Oct3/4, Klf4, Sox2, c-Myc (here, Sox2 is replaceable with Soxl, Sox3, Sox15, Soxl7 or Sox18; Klf4 is replaceable with Klfl, Klf2 or KlfS; c-Myc is replaceable with T58A (active mutant), N-Myc or L-Myc)
(2) Oct3/4, Klf4, Sox2
(3) Oct3/4, Klf4, c-Myc
(4) Oct3/4, Sox2, Nanog, Lin28
(5) Oct3/4, Klf4, c-Myc, Sox2, Nanog, Lin28
(6) Oct3/4, Klf4, Sox2, bFGF
(7) Oct3/4, Klf4, Sox2, SCF
(8) Oct3/4, Klf4, c-Myc, Sox2, bFGF
(9) Oct3/4, Klf4, c-Myc, Sox2, SCF

Among these combinations, when the obtained iPS cell is to be used for therapeutic purposes, a combination of 4 factors of (1), a combination of 3 factors of (2), and 6 factors of (5) are preferable. Above all, a combination of 4 factors of (1), and a combination of 3 factors of (2) are more preferable. On the other hand, when the iPS cell is not to be used for therapeutic purposes (e.g., used as an investigational tool for drug discovery screening and the like), 4 factors of Oct3/4, Klf4, Sox2, and c-Myc, or 5 factors of further including Lin28 or Nanog is preferred.

iPS cell is preferably used for autologous transplantation.

Pluripotent stem cells whose chromosomal genes have been altered using known genetic engineering techniques can also be used in the present invention. The pluripotent stem cell may be a cell that can be identified to have reached a corresponding differentiation stage, by using the expression of a marker gene (e.g., fluorescent protein such as GFP) as an index, which is achieved by knocking in the marker gene in-frame according to a known method to a gene encoding a differentiation marker.

As the pluripotent stem cells, pluripotent stem cells of, for example, homoiothermic animals, preferably mammals, can be used. Examples of the mammal include experimental animals such as rodents (e.g., mouse, rat, hamster, guinea pig, and the like), and rabbit and the like, domestic animals such as swine, bovine, goat, horse and sheep, pets such as dog and cat and the like, and the like, primates such as human, monkey, orangutan and chimpanzee, and the like, and the like. The pluripotent stem cell is preferably a pluripotent stem cell of rodents (mouse, rat, etc.) or primates (human, etc.), most preferably a human pluripotent stem cell.

Pluripotent stem cells can be maintenance cultured by a method known per se. For example, from the viewpoint of clinical application, pluripotent stem cells are preferably maintained by serum-free culture using a serum replacement such as Knockout^{™} Serum Replacement (KSR) and the like, or feeder-free cell culture.

Pluripotent stem cells used in the present invention are preferably isolated. The "isolation" means that an operation has been performed to remove factors other than the desired cells and components, and that the naturally occurring state has passed. The purity (percentage of human pluripotent stem cells to the total number of cells) of the "isolated human pluripotent stem cells" is generally 70% or more, preferably 80% or more, more preferably 90% or more, further preferably 99% or more, most preferably 100%.

### (2) Formation of pluripotent stem cell aggregate

The method for preparing aggregates of pluripotent stem cells is not particularly limited, and the dispersed pluripotent stem cells may be cultured under non-adhesive conditions (i.e., suspension culture) in a culture container, or may be cultured under adhesive conditions (i.e., adherent culture).

In one preferable embodiment, dispersed pluripotent stem cells are cultured in a culture vessel under non-adhesive conditions (i.e., suspension culture), and a plurality of pluripotent stem cells are assembled to form aggregates, whereby aggregates of pluripotent stem cells can be obtained.

A culture vessel used for the aggregate formation is not particularly limited, and, for example, flask, tissue culture flask, dish, petri dish, tissue culture dish, multidish, microplate, microwell plate, micropore, multiplate, multiwell plate, chamber slide, petri dish, tube, tray, culture bag, and roller bottle can be mentioned. To enable culture under non-adhesive conditions, the culture vessel is preferably non-adhesive to cells. As the cell non-adhesive culture vessel, a culture vessel whose surface has been artificially treated to be non-adhesive to cells, a culture vessel that has not been artificially treated (for example, coating treatment with extracellular matrix, etc.) for the purpose of improving adhesion to cells, or the like can be used.

The medium used for aggregate formation can be prepared using a medium used for culturing mammalian cells as a basal medium. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium=1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. The serum-free medium means a medium free of an unadjusted or unpurified serum, and a medium containing a purified blood-derived component or a purified animal tissue-derived component (e.g., growth factor) mixed therein is considered to fall under the serum-free medium. To avoid contamination with chemically undetermined components, a serum-free medium is preferably used in the present invention.

The medium used for aggregate formation may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for aggregate formation may contain other additives as long as it does not adversely affect induction of differentiation of pluripotent stem cells into pituitary gland or a partial tissue thereof, or a progenitor tissue thereof. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In addition, the medium used for aggregate formation may be the medium used in the first culture step, which is described later.

In the formation of pluripotent stem cell aggregates, pluripotent stem cells are first collected from the passage culture, and dispersed to a single cell state or a state close thereto. Dispersion of pluripotent stem cells is performed using an appropriate cell dissociation solution. As the cell dissociation solution, EDTA; trypsin, collagenase IV, protein degrading enzyme such as metalloprotease and the like, and the like can be used alone or in an appropriate combination. Among them, those with low cytotoxicity are preferred. As such cell dissociation solution, for example, commercially available products such as DISPASE (EIDIA Co., Ltd), TrypLE (Invitrogen), Accutase (MILLIPORE), and the like are available. The dispersed pluripotent stem cells are suspended in the above-mentioned medium.

In order to suppress cell death of pluripotent stem cells (particularly, human pluripotent stem cells) induced by dispersing, it is preferable to add an inhibitor of Rho-associated coiled-coil kinase (ROCK) from the start of culture (JP-A-2008-99662 ). A ROCK inhibitor is added, for example, within 15 days, preferably within 10 days, and more preferably within 6 days, from the start of culture. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for suspension culture is a concentration that can suppress cell death of pluripotent stem cells induced by dispersing. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period.

A suspension of the dispersed pluripotent stem cells is seeded in the above-mentioned culture vessel and the dispersed pluripotent stem cells are cultured in the culture vessel under non-adhesive conditions, whereby a plurality of pluripotent stem cells are assembled to form aggregates. At this time, by seeding the dispersed pluripotent stem cells in a relatively large culture vessel such as a 10 cm dish, multiple aggregates of pluripotent stem cells can be simultaneously formed in one culture compartment. In this case, however, the size of each aggregate and the number of pluripotent stem cells contained inside vary greatly. Due to the variation, the degree of differentiation from pluripotent stem cells into pituitary gland, a partial tissue thereof, or a progenitor tissue thereof differs between aggregates, which in turn causes a decrease in the efficiency of differentiation induction. Therefore, it is preferable to rapidly coagulate dispersed pluripotent stem cells to form one aggregate in one culture compartment. As the methods for rapidly coagulating such dispersed pluripotent stem cells, for example, the following methods can be mentioned:
1) A method of confining dispersed pluripotent stem cells in a culture compartment of relatively small volume (e.g., 1 ml or less, 500 ul or less, 200 ul or less, 100 ul or less) and forming one aggregate in the compartment. Preferably, after confining the dispersed pluripotent stem cells, the culture compartment is left standing. Examples of the culture compartment include, but are not limited to, multiwell plates (384 wells, 192 wells, 96 wells, 48 wells, 24 wells, etc.), wells in micropore, chamber slide, and the like, tube, medium droplets in the hanging drop method, and the like. The dispersed pluripotent stem cells confined in the compartment are forced to deposit in one location by gravity, or adhere to each other to form one aggregate per one culture compartment. The shape of the bottom of multiwell plate, micropore, chamber slide, tube, and the like is preferably U-bottomed or V-bottomed so that the dispersed pluripotent stem cells can easily deposit in one place.
2) A method of placing the dispersed pluripotent stem cells in a centrifugation tube and centrifuging them to cause precipitation of the pluripotent stem cells in one location, thereby forming one aggregate in the tube.

The number of pluripotent stem cells to be seeded in one culture compartment is not particularly limited as long as one aggregate is formed per culture compartment, and the method of the present invention can induce the differentiation of pluripotent stem cells into pituitary gland or a partial tissue thereof, or a progenitor tissue thereof in the aggregate. Generally about 1×10³ to about 5×10⁴, preferably about 1×10³ to about 2×10⁴, more preferably about 2×10³ to about 1.2×10⁴, pluripotent stem cells are seeded per culture compartment. By causing rapid coagulation of the pluripotent stem cells, one cell aggregate composed of generally about 1×10³ to about 5×10⁴, preferably about 1×10³ to about 2×10⁴, more preferably about 2×10³ to about 1.2×10⁴, pluripotent stem cells can be formed per culture compartment.

The time up to aggregate formation can be determined as appropriate within the range where one aggregate is formed per culture compartment, and the method of the present invention can induce the differentiation of pluripotent stem cells into pituitary gland or a partial tissue thereof, or a progenitor tissue thereof in the aggregate. This time is preferably as short as possible because efficient induction of desired differentiation into pituitary gland or a partial tissue thereof, or a progenitor tissue thereof can be expected by shortening this time. Preferably, aggregates of pluripotent stem cells are formed within 24 hours, more preferably within 12 hours, still more preferably within 6 hours, and most preferably within 2 to 3 hours. The time up to aggregate formation can be appropriately adjusted by those of ordinary skill in the art by adjusting tools for causing aggregation of cells, centrifugation conditions, and the like.

In addition, other culture conditions such as culture temperature, CO₂ concentration, and the like during aggregate formation can be appropriately set. The culture temperature is not particularly limited and is, for example, about 30 - 40°C, preferably about 37°C. The CO₂ concentration is, for example, about 1 to 10%, preferably about 5%.

Furthermore, by preparing a plurality of culture compartments under the same culture conditions and forming one aggregate of pluripotent stem cells in each culture compartment, a population of qualitatively uniform aggregates of pluripotent stem cells can be obtained. The qualitative uniformity of aggregates of pluripotent stem cells can be evaluated based on the size, the number of cells, macroscopic morphology, microscopic morphology and uniformity thereof by tissue staining analysis, expression of differentiation and undifferentiated markers and uniformity thereof, regulation of the expression of differentiation markers and synchronism thereof, of the aggregates, reproducibility of differentiation efficiency between aggregates, and the like. In one embodiment, the population of pluripotent stem cell aggregates used in the method of the present invention has a uniform number of pluripotent stem cells contained in the aggregates. Regarding a specific parameter, when a population of pluripotent stem cell aggregates is "uniform," it means that 90% or more of the aggregates in the entire aggregate population are within the range of mean±10%, preferably within the range of mean ± 5%, of the parameter in the aggregate population.

### (3) Induction of adenohypophysis and/or progenitor tissue thereof

In one embodiment, a cell aggregate before cell separation can be obtained by a method including suspension culturing the aggregates of pluripotent stem cells in a medium containing a bone morphogenic factor signal transduction pathway activating substance and a Sonic hedgehog (Shh) signal pathway agonist.

In the present invention, the adenohypophysis refers to a tissue containing at least one type of pituitary hormone-producing cell of the anterior or intermediate pituitary gland. Examples of the pituitary hormone-producing cell include cells constituting the anterior lobe such as growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, luteinizing hormone (LH)-producing cells, and the like; and cells constituting the intermediate lobe such as melanocyte stimulation hormone (MSH)-producing cells and the like. These pituitary hormone-producing cells can express EpCAM as a marker in addition to the expression of pituitary hormone specific to each cell type. In one embodiment, adenohypophysis contains at least one type, preferably two or more types (2, 3, 4, 5, or 6 types) of pituitary hormone-producing cells selected from the group consisting of GH-producing cells, PRL-producing cells, ACTH-producing cells, TSH-producing cells, FSH-producing cells, and LH-producing cells. In a further embodiment, adenohypophysis contains at least one type, preferably two types, more preferably 3 types, of pituitary hormone-producing cells selected from the group consisting of GH-producing cells, PRL-producing cells, and ACTH-producing cells.

In the present invention, the tissue refers to the structure of a cell population that has a structure in which multiple types of cells with different morphologies and properties are three-dimensionally configured in a certain pattern.

As the progenitor tissue of adenohypophysis, pituitary placode, Rathke's pouch, and the like can be mentioned. The pituitary placode refers to a thickened structure that is formed in the area of the epidermal ectoderm (oral ectoderm) during embryogenesis and can express at least pituitary progenitor cell markers EpCAM, and Lhx3 or Pitxl, preferably all of EpCAM, Lhx3, and Pitxl. The Rathke's pouch refers to a sac-like structure formed by invagination of pituitary placode. The Rathke's pouch can also, like pituitary placode, express at least pituitary progenitor cell markers EpCAM, and Lhx3 or Pitxl, preferably all of EpCAM, Lhx3, and Pitxl. As pituitary progenitor cell markers other than the above, the expression of Isll/2, Cytokeratin, and the like may be further confirmed. In the present specification, a cell capable of expressing at least pituitary progenitor cell markers EpCAM, and Lhx3 or Pitxl is a pituitary progenitor cell, and this progenitor cell can preferably express all of EpCAM, Lhx3, and Pitxl. In the present specification, the pituitary progenitor cell is also referred to as a progenitor cell of pituitary hormone-producing cells.

As the "cell aggregate before cell separation" in the present specification, for example, a cell aggregate positive for at least one marker selected from LHX3, NKX2.l, PITX1, and ACTH can be mentioned.

As the "cell aggregate before cell separation", for example, a LHX3, NKX2.l, PITXl, and ACTH-positive cell aggregate can be mentioned.

The cell aggregate before cell separation to be used in the separation method and the production method of the present invention can be produced by the following method. To be specific, the method includes suspension culturing pluripotent stem cell aggregates in a medium containing a bone morphogenic factor signal transduction pathway activation substance and an Shh signal pathway agonist to obtain cell aggregates containing hypothalamic neuroepithelial tissue and epidermal ectoderm (including oral ectoderm) (hereinafter, sometimes to be simply abbreviated as epidermal ectoderm) (the first culture step), and further suspension culturing the obtained cell aggregates containing hypothalamic neuroepithelial tissue and epidermal ectoderm (including oral ectoderm) in a medium containing a bone morphogenic factor signal transduction pathway activation substance and an Shh signal pathway agonist to obtain cell aggregates containing 1) hypothalamic neuroepithelial tissue and 2) pituitary placode and/or Rathke's pouch (the second culture step). The first culture step induces differentiation of pluripotent stem cells into hypothalamic neuroepithelial tissue and epidermal ectoderm (including oral ectoderm), and further differentiation of epidermal ectoderm region (oral ectoderm) into pituitary placode and/or Rathke's pouch is induced by subjecting the obtained cell aggregates containing hypothalamic neuroepithelial tissue and epidermal ectoderm (including oral ectoderm) to the second culture step.

In the separation method and the production method of the present invention, the cell aggregate before cell separation may be a cell aggregate obtained by the second culture step.

### (3.1) The first culture step

In the first culture step, pluripotent stem cell aggregates are cultured in suspension in a medium containing a bone morphogenic factor signal transduction pathway activation substance and a Shh signal pathway agonist.

That the pluripotent stem cell aggregates are "cultured in suspension" means that pluripotent stem cell aggregates are cultured in a medium under a condition of non-adhesive to the culture vessel. This enables efficient induction of adenohypophysis or a progenitor tissue thereof, which has conventionally been difficult to achieve.

The medium used for suspension culture contains a bone morphogenic factor signal transduction pathway activation substance and a Shh signal pathway agonist. The differentiation of the pluripotent stem cells into hypothalamic neuroepithelial tissue and epidermal ectoderm is induced by the action of the bone morphogenic factor signal transduction pathway activation substance and the Shh signal pathway agonist.

In the present invention, the bone morphogenic factor signal transduction pathway activation substance means any substance that activates a pathway in which signals are transduced by the binding of a bone morphogenic factor and a receptor. Examples of the bone morphogenic factor signal transduction pathway activation substance include BMP2, BMP4, BMP7, GDF5, and the like. A preferred bone morphogenic factor signal transduction pathway activation substance is BMP4. While BMP4 is mainly described in the following, the bone morphogenic factor signal transduction pathway activation substance used in the present invention is not limited to BMP4. BMP4 is a known cytokine, and the amino acid sequence thereof is also known. The BMP4 used in the present invention is a mammalian BMP4. Examples of the mammal include rodents such as mouse, rat, hamster, guinea pig and the like, experimental animals such as rabbit and the like, domestic animals such as swine, bovine, goat, horse, sheep, and the like, pets such as dog, cat and the like, primates such as human, monkey, orangutan, chimpanzee, and the like, and the like. BMP4 is preferably a BMP4 of rodents (mouse, rat etc.) or primates (human etc.), most preferably human BMP4. Human BMP4 means that BMP4 has the amino acid sequence of BMP4 that humans naturally express in vivo. Representative amino acid sequences of human BMP4 are, for example, NCBI accession numbers NP_001193.2 (updated June 15, 2013), NP_570911.2 (updated June 15, 2013), and NP_570912.2 (updated June 15, 2013), amino acid sequences (mature human BMP4 amino acid sequences) obtained by removing N terminal signal sequence (1-24) from each of these amino acid sequences, and the like.

In the present invention, the Shh signal pathway agonist is not particularly limited as long as it can enhance signal transduction mediated by Shh. Examples of the Shh signal pathway agonist include, but are not limited to, proteins belonging to the Hedgehog family and fragments thereof (e.g., Shh, Ihh, Shh(C24II) N-Terminal, Shh(C25II) N-Terminal), Shh receptor, Shh receptor agonist, Purmorphamine, Smoothened - Agonist (SAG) (3-Chloro-N-[trans-4-(methylamino)cyclohexyl]-N-[[3-(4-pyridinyl)phenyl]methyl]-benzo[b]thiophene-2-carboxamide), and the like. Among these, SAG is preferred.

A preferred combination of the bone morphogenic factor signal transduction pathway activation substance and the Shh signal pathway agonist is BMP4 and SAG.

The concentration of the bone morphogenic factor signal transduction pathway activation substance in the medium can be appropriately determined within the range where differentiation from pluripotent stem cells to hypothalamic neuroepithelial tissue and epidermal ectoderm can be induced. When BMP4 is used as the bone morphogenic factor signal transduction pathway activation substance, its concentration is generally 0.01 nM or more, preferably 0.1 nM or more, more preferably 1 nM or more. The upper limit is not particularly set as long as differentiation into hypothalamic neuroepithelial tissue and epidermal ectoderm is not adversely affected. From the aspect of culture cost, it is generally 1000 nM or less, preferably 100 nM or less, more preferably 10 nM or less. In one embodiment, the concentration of BMP4 in the medium is generally 0.01 to 1000 nM, preferably 0.1 to 100 nM, more preferably 1 to 10 nM (e.g., 5 nM). Exogenous bone morphogenic factor signal transduction pathway activation substance particularly contributes to 1) active formation of epidermal ectoderm and 2) induction of differentiation into neuroepithelial tissue of the hypothalamus, not of the cerebrum, in cell aggregates. Thus, it is contained in the medium at concentrations capable of achieving these effects.

The bone morphogenic factor signal transduction pathway activation substance may not be contained in the medium for the entire period of the first culture step. For example, the bone morphogenic factor signal transduction pathway activation substance may not be added to the medium 2 to 4 days (e.g., 3 days) from the start of the suspension culture of pluripotent stem cell aggregates, and thereafter the bone morphogenic factor signal transduction pathway activation substance may be added to the medium.

The concentration of the Shh signal pathway agonist in the medium can be appropriately determined within the range where differentiation from pluripotent stem cells to hypothalamic neuroepithelial tissue and epidermal ectoderm can be induced. When SAG is used as the Shh signal pathway agonist, its concentration is generally 1 nM or more, preferably 10 nM or more, more preferably 100 nM or more. The upper limit is not particularly set as long as differentiation into hypothalamic neuroepithelial tissue and epidermal ectoderm is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM). Exogenous Shh signal pathway agonist particularly contributes to the induction of differentiation into neuroepithelial tissue of the hypothalamus (preferably ventral hypothalamus), not of the neural retina, in cell aggregates. Thus, it is contained in the medium at a concentration capable of achieving this effect.

The Shh signal pathway agonist may not be contained in the medium for the entire period of the first culture step. For example, the Shh signal pathway agonist may not be added to the medium 5 to 7 days (e.g., 6 days) from the start of the suspension culture of pluripotent stem cell aggregates, and thereafter the Shh signal pathway agonist may be added to the medium.

In one embodiment, pluripotent stem cell aggregates are cultured in suspension for 2 to 4 days in a medium not containing a bone morphogenic factor signal transduction pathway activation substance or an Shh signal pathway agonist, then the obtained aggregates are cultured in suspension for 2 to 4 days in a medium containing a bone morphogenic factor signal transduction pathway activation substance but not containing an Shh signal pathway agonist, and further the obtained aggregates are cultured in suspension in a medium containing a bone morphogenic factor signal transduction pathway activation substance and an Shh signal pathway agonist until hypothalamic neuroepithelial tissue and epidermal ectoderm are induced.

Bone morphogenic factor signal transduction pathway activation substances such as BMP4 and the like used in the present invention are preferably isolated. The "isolation" means that an operation has been performed to remove factors other than the desired components and cells, and that the naturally occurring state has passed. Therefore, the "isolated protein X" does not include endogenous protein X produced from a cell or tissue to be cultured. The purity (percentage of weight of protein X to the total protein weight) of the "isolated protein X" is generally 70% or more, preferably 80% or more, more preferably 90% or more, further preferably 99% or more, most preferably 100%. The isolated bone morphogenic factor signal transduction pathway activation substance contained in the medium used for suspension culture is exogenously added to the medium. Therefore, in one embodiment, the present invention includes a step of exogenously adding an isolated bone morphogenetic factor signal transduction pathway activation substance to the medium used in the first culture step.

In order to suppress cell death of pluripotent stem cells (particularly, human pluripotent stem cells) induced by dispersing, it is preferable to add an inhibitor of Rho-associated coiled-coil kinase (ROCK) to the medium to be used in the first culture step from the start of culture ( JP-A-2008-99662 ). A ROCK inhibitor is added, for example, within 15 days, preferably within 10 days, and more preferably within 6 days, from the start of culture. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for suspension culture is a concentration that can suppress cell death of pluripotent stem cells induced by dispersing. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period.

The medium used for suspension culture of cell aggregates can be prepared using a medium used for culturing mammalian cells as a basal medium. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium-1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of pluripotent stem cells into hypothalamic neuroepithelial tissue and epidermal ectoderm. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into hypothalamic neuroepithelial tissue and epidermal ectoderm, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

Other culture conditions such as culture temperature, CO₂ concentration, O₂ concentration, and the like in suspension culture of cell aggregates can be appropriately set. The culture temperature is, for example, about 30 - 40°C, preferably about 37°C. The CO₂ concentration is, for example, about 1 to 10%, preferably about 5%. The O₂ concentration is, for example, about 20%.

In a preferred embodiment, a population of qualitatively uniform aggregates of pluripotent stem cells is cultured in suspension in a medium containing a bone morphogenic factor signal transduction pathway activation substance and an Shh signal pathway agonist. Using a population of qualitatively uniform aggregates of pluripotent stem cells, the difference in the degree of differentiation into adenohypophysis or a progenitor tissue thereof between aggregates can be minimized and the efficiency of the desired differentiation induction can be improved. The suspension culture of a population of qualitatively uniform aggregates of pluripotent stem cells includes the following embodiments.
1) A plurality of culture compartments are prepared and a population of qualitatively uniform aggregates of pluripotent stem cells is seeded such that one pluripotent stem cell aggregate is contained in one culture compartment (e.g., one pluripotent stem cell aggregate is placed in each well of 96 well plate). Then, in each culture compartment, one pluripotent stem cell aggregate is cultured in suspension in a medium containing a bone morphogenic factor signal transduction pathway activation substance and a Shh signal pathway agonist.
2) Populations of qualitatively uniform aggregates of pluripotent stem cells are seeded in one culture compartment such that a plurality of pluripotent stem cell aggregates are contained in one culture compartment (e.g., plural pluripotent stem cell aggregates are placed in a 10 cm dish). Then, in the culture compartment, a plurality of pluripotent stem cell aggregates are cultured in suspension in a medium containing a bone morphogenic factor signal transduction pathway activation substance and an Shh signal pathway agonist.

Throughout the method of the present invention, any embodiment of the above-mentioned 1) and 2) may be adopted, and the embodiment may be changed during the culture. (From embodiment 1) to embodiment 2), or from embodiment 2) to embodiment 1)). In one embodiment, the first culture step employs the embodiment 1), and the second culture step employs the embodiment 2).

The first culture step is conducted for a period of time sufficient to induce differentiation of pluripotent stem cells into hypothalamic neuroepithelial tissue and epidermal ectoderm. The differentiation of pluripotent stem cells into hypothalamic neuroepithelial tissue and epidermal ectoderm can be detected by, for example, RT-PCR and immunohistochemistry using marker-specific antibodies in hypothalamic neuroepithelial tissue and epidermal ectoderm. For example, the step is conducted until 10% or more, preferably 30% or more, more preferably 50% or more, of the cell aggregates in the culture contain hypothalamic neuroepithelial tissue and epidermal ectoderm. The culture period may vary according to the animal species of the pluripotent stem cells, and the kind of the bone morphogenic factor signal transduction pathway activation substance and the Shh signal pathway agonist and cannot be specified. When a human pluripotent stem cell is used, the first culture step is, for example, generally 15 to 20 days (e.g., 18 days).

By performing the first culture step, cell aggregates containing hypothalamic neuroepithelial tissue and epidermal ectoderm (including oral ectoderm) can be obtained.

The hypothalamic neuroepithelial tissue refers to a neuroepithelial tissue that expresses a hypothalamic marker, can differentiate into a cell constituting hypothalamus (e.g., neuron, glial cell, and the like), and contains a hypothalamic progenitor cell having self-renewal potential, neurons and glial cells that have lost self-renewal potential due to differentiation, and the like. In the present specification, cells constituting the hypothalamus are also referred to as "hypothalamic cells," as the cells, for example, hypothalamic progenitor cells, neurons, glial cells and the like can be mentioned. The ratio of cells consisting of the hypothalamic nerve epithelium, hypothalamic progenitor cells and differentiated cells thereof, in the hypothalamic neuroepithelial tissue depends on the degree of differentiation. It has been confirmed that many of the hypothalamic progenitor cells differentiate into hypothalamic neurons in cell aggregates obtained by culturing pluripotent stem cells for a long term until they differentiate into adenohypophysis (Cell Reports, 30, 18-24, January 7, 2020). The hypothalamus includes ventral hypothalamus and dorsal hypothalamus. As the hypothalamic marker, NKx2.1 (ventral hypothalamic marker), Pax6 (dorsal hypothalamic marker), and the like can be mentioned. In one embodiment, the ventral hypothalamic neuroepithelial tissue is an Rx-positive, Chx10-negative, Pax6-negative, and Nkx2.1-positive neuroepithelial tissue. In one embodiment, the dorsal hypothalamic neuroepithelial tissue is an Rx-positive, Chx10-negative, Nkx2.1-negative, and Pax6-positive neuroepithelial tissue. The hypothalamic neuroepithelial tissue contained in the cell aggregate obtained in the first culture step is preferably a ventral hypothalamic neuroepithelial tissue. EpCAM is not expressed in any of the above-mentioned hypothalamic neuroepithelial tissues, which means that EpCAM is not expressed in all cells that constitute the hypothalamic neuroepithelial tissue (not only hypothalamic progenitor cells, but also differentiated neurons and glial cells that have lost self-renewal potential).

The epidermal ectoderm is an ectodermal cell layer formed on the surface layer of the embryo during embryogenesis. As an epidermal ectoderm marker, pan-cytokeratin can be mentioned. The epidermal ectoderm can be generally differentiated into anterior pituitary gland, skin, oral epithelium, tooth enamel, dermal gland, and the like. In one embodiment, the epidermal ectoderm is an E-cadherin-positive and pan-cytokeratin-positive cell layer.

Preferably, in the cell aggregates obtained in the first culture step, the hypothalamic neuroepithelial tissue occupies the interior of the cell aggregates, and a single layer of epidermal ectodermal cells constitutes the surface of the cell aggregates. The epidermal ectoderm may contain a thickened epidermal placode in a part thereof.

### (3.2) the second culture step

In the second culture step, the cell aggregates obtained in the first culture step and containing hypothalamic neuroepithelial tissue and epidermal ectoderm (including oral ectoderm) are further cultured in suspension in a medium containing a bone morphogenic factor signal transduction pathway activation substance and a Shh signal pathway agonist to give cell aggregates containing 1) hypothalamic neuroepithelial tissue, and 2) pituitary placode and/or Rathke's pouch. By the action of the bone morphogenic factor signal transduction pathway activation substance and the Shh signal pathway agonist, the differentiation of epidermal ectoderm into pituitary placode and/or Rathke's pouch is induced.

The definitions of the bone morphogenic factor signal transduction pathway activation substance and the Shh signal pathway agonist are as described in the first culture step.

Preferably, the bone morphogenic factor signal transduction pathway activation substance to be used in the second culture step is the same as that in the first culture step and is BMP4. Preferably, the Shh signal pathway agonist to be used in the second culture step is the same as that in the first culture step and is SAG.

A preferred combination of the bone morphogenic factor signal transduction pathway activation substance and the Shh signal pathway agonist is BMP4 and SAG.

The concentration of the bone morphogenic factor signal transduction pathway activation substance in the medium can be appropriately determined within the range where differentiation from epidermal ectoderm to pituitary placode and/or Rathke's pouch can be induced. When BMP4 is used as the bone morphogenic factor signal transduction pathway activation substance, its concentration is generally 0.01 nM or more, preferably 0.1 nM or more, more preferably 1 nM or more. The upper limit is not particularly set as long as differentiation of epidermal ectoderm into pituitary placode and/or Rathke's pouch is not adversely affected. From the aspect of culture cost, it is generally 1000 nM or less, preferably 100 nM or less, more preferably 10 nM or less. In one embodiment, the concentration of BMP4 in the medium is generally 0.01 to 1000 nM, preferably 0.1 to 100 nM, more preferably 1 to 10 nM (e.g., 5 nM). The concentration of the bone morphogenic factor signal transduction pathway activation substance may be varied during the period of addition. For example, the aforementioned concentration may be used at the start of the second culture step, and the concentration may be reduced stepwise to half every 2 to 4 days.

The concentration of the Shh signal pathway agonist in the medium can be appropriately determined within the range where differentiation from epidermal ectoderm into pituitary placode and/or Rathke's pouch can be induced. When SAG is used as the Shh signal pathway agonist, its concentration is generally 1 nM or more, preferably 10 nM or more, more preferably 100 nM or more. The upper limit is not particularly set as long as differentiation into pituitary placode and/or Rathke's pouch is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM).

In a preferred embodiment, the medium used in the second culture step contains FGF2. FGF2 promotes differentiation of epidermal ectoderm into pituitary placode.

FGF2 is a known cytokine also called a basic fibroblast growth factor (bFGF), and the amino acid sequence thereof is also known. The FGF2 used in the present invention is generally a mammalian FGF2. As the mammal, those mentioned above can be recited. Since FGF2 has cross-reactivity among many mammalian species, any mammalian FGF2 may be used as long as the purpose of the present invention can be achieved. Preferably, mammalian FGF2 of the same species as the cells to be cultured is used. For example, FGF2 of rodents (mouse, rat, etc.) or primates (human, etc.) is used. As used herein, mouse FGF2 means that FGF2 has the amino acid sequence of FGF2 that mice naturally express in vivo. Other protein and the like in the present specification are also interpreted similarly. Representative amino acid sequences of mouse FGF2 are, for example, NCBI accession number NP_032032.1 (updated February 18, 2014), an amino acid sequence (mature mouse FGF2 amino acid sequences) obtained by removing N terminal signal sequence (1-9) from this amino acid sequence, and the like. Representative amino acid sequences of human FGF2 are, for example, NCBI accession number NP_001997.5 (updated February 18, 2014) and the like.

The concentration of FGF2 in the medium is not particularly limited as long as it can promote differentiation of epidermal ectoderm into pituitary placode. It is generally 1 ng/ml or more, preferably 10 ng/ml or more. The upper limit of the FGF2 concentration is not particularly set as long as differentiation into pituitary placode and/or Rathke's pouch is not adversely affected. From the aspect of culture cost, it is generally 1000 ng/ml or less, preferably 500 ng/ml or less. In one embodiment, the concentration of FGF2 in the medium is generally 1 to 1000 ng/ml, preferably 10 to 100 ng/ml.

Bone morphogenic factor signal transduction pathway activation substance such as BMP4 and the like and FGF2 used in the present invention are preferably isolated. The isolated bone morphogenic factor signal transduction pathway activation substance and isolated FGF2 contained in the medium used in the second culture step are exogenously added to the medium. Therefore, in one embodiment, the present invention includes a step of exogenously adding an isolated bone morphogenetic factor signal transduction pathway activation substance (and optionally isolated FGF2) to the medium used in the second culture step.

The medium used in the second culture step can be prepared using a medium used for culturing mammalian cells as a basal medium, similar to the medium used in the first culture step. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium=1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of epidermal ectoderm into pituitary placode and/or Rathke's pouch. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into pituitary placode and/or Rathke's pouch, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

The suspension culture in the second culture step is preferably performed under high oxygen partial pressure conditions. Further suspension culture of cell aggregates containing hypothalamic neuroepithelial tissue and epidermal ectoderm under high oxygen partial pressure conditions allows oxygen to reach the inside of the cell aggregates and achieves maintenance culture of the cell aggregates for a long period of time, which in turn enables efficient induction of differentiation into pituitary placode and/or Rathke's pouch.

The high oxygen partial pressure conditions mean oxygen partial pressure conditions above the oxygen partial pressure in air (20%). The oxygen partial pressure in the second culture step is, for example, 30 to 60%, preferably 35 to 60%, more preferably 38 to 60%.

Other culture conditions such as culture temperature, CO₂ concentration and the like in the second culture step can be appropriately set. The culture temperature is, for example, about 30 to 40°C, preferably about 37°C. The CO₂ concentration is, for example, about 1 to 10%, preferably about 5%.

The second culture step is conducted for a period of time sufficient to induce differentiation of epidermal ectoderm into pituitary placode and/or Rathke's pouch. By performing the second culture step, pituitary placode can be formed in the epidermal ectoderm (more specifically, in the oral ectoderm). In addition, a part or all of the pituitary placode invaginates toward the inside of the cell aggregate (i.e., adjacent hypothalamic neuroepithelium) to optionally form Rathke's pouch. Differentiation of epidermal ectoderm into pituitary placode and/or Rathke's pouch essentially requires interaction between epidermal ectoderm and hypothalamic neuroepithelial tissue (preferably, ventral hypothalamic neuroepithelial tissue). In the present invention, hypothalamic neuroepithelial tissue and epidermal ectoderm are simultaneously formed in the cell aggregates in the first culture step, hypothalamic neuroepithelial tissue occupies the inside of the cell aggregates in a preferred embodiment, and the cells of the epidermal ectoderm in a single layer constitute the surface of the cell aggregates. As a result, a good interaction between the adjacent epidermal ectoderm and hypothalamic neuroepithelial tissue becomes possible in the cell aggregates, and the process of self organization of pituitary gland during embryonic development, such as formation of pituitary placode in epidermal ectoderm, invagination of pituitary placode, formation of Rathke's pouch, and the like can be reproduced in vitro. Differentiation into pituitary placode and/or Rathke's pouch can be confirmed, for example, by detecting the formation of a pituitary progenitor cell marker-positive placode or pouch-like structure, by immunohistochemistry using specific antibodies against pituitary progenitor cell markers (e.g. EpCAM, Pitx1, Lhx3, etc.). For example, the second culture step is conducted until 10% or more, preferably 30% or more, more preferably 50% or more, of the cell aggregates in the culture contain pituitary placode and/or Rathke's pouch. The culture period may vary according to the animal species of the pluripotent stem cells, and the kind of the bone morphogenic factor signal transduction pathway activation substance and the Shh signal pathway agonist and cannot be specified. When a human pluripotent stem cell is used, the period of the second culture step is, for example, generally not less than 6 days, for example, 6 to 12 days.

By performing the second culture step, cell aggregates containing 1) hypothalamic neuroepithelial tissue, and 2) pituitary placode and/or Rathke's pouch can be obtained. In the separation method and production method of the present invention, the cell aggregate before cell separation may be a cell aggregate obtained in the second culture step.

### (3.3) The third culture step

Further suspension culture of the cell aggregates containing 1) hypothalamic neuroepithelial tissue, and 2) pituitary placode and/or Rathke's pouch, obtained in the second culture step, in a medium containing an Shh signal pathway agonist affords cell aggregates containing adenohypophysis (third culture step). By the third culture step, differentiation of pituitary placode and/or Rathke's pouch into pituitary hormone-producing cells is induced, pituitary hormone-producing cells are produced in the pituitary placode and/or Rathke's pouch, and adenohypophysis can be formed.

In the separation method and the production method of the present invention, the cell aggregate before cell separation may be a cell aggregate obtained by the third culture step.

The definitions of the Shh signal pathway agonist are as described in the first culture step.

Preferably, the Shh signal pathway agonist to be used in the third culture step is the same as that in the first and the second culture steps and is SAG.

The concentration of the Shh signal pathway agonist in the medium can be appropriately determined within the range where differentiation from pituitary placode and/or Rathke's pouch into pituitary hormone-producing cells can be induced. When SAG is used as the Shh signal pathway agonist, its concentration is generally 1 nM or more, preferably 10 nM or more, more preferably 100 nM or more. The upper limit is not particularly set as long as differentiation into pituitary hormone-producing cells is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM).

In a preferred embodiment, the medium used in the third culture step contains FGF2. FGF2 promotes differentiation of pituitary placode and/or Rathke's pouch into pituitary hormone-producing cells.

The definition of FGF2 is as described in the second culture step.

The concentration of FGF2 in the medium is not particularly limited as long as it can promote differentiation of pituitary placode and/or Rathke's pouch into pituitary hormone-producing cells. It is generally 1 ng/ml or more, preferably 10 ng/ml or more. The upper limit of the FGF2 concentration is not particularly set as long as differentiation into pituitary hormone-producing cells is not adversely affected. From the aspect of culture cost, it is generally 1000 ng/ml or less, preferably 500 ng/ml or less. In one embodiment, the concentration of FGF2 in the medium is generally 1 to 1000 ng/ml, preferably 10 to 100 ng/ml.

In a preferred embodiment, the medium used in the third culture step contains a Notch signal inhibitor. A Notch signal inhibitor promotes differentiation of pituitary placode and/or Rathke's pouch into pituitary hormone-producing cells (particularly, ACTH-producing cells). The Notch signal inhibitor increases the expression of transcription factor Tbxl9 that upregulates ACTH production.

The Notch signal inhibitor is not particularly limited as long as it can suppress signal transduction mediated by Notch. Examples of the Notch signal inhibitor include gamma secretase inhibitors such as DAPT (N-[N-(3,5-difluorophenacetyl)-1-alanyl]-S-phenylglycine t-butyl ester), DBZ, MDL28170, and the like. Among these, DAPT is preferred.

The concentration of the Notch signal inhibitor in the medium is not particularly limited as long as it can promote differentiation of pituitary placode and/or Rathke's pouch into pituitary hormone-producing cells (particularly, ACTH-producing cells). For example, in the case of DAPT, it is generally 0.1 µM or more, preferably 1 µM or more. The upper limit of the DAPT concentration is not particularly set as long as differentiation into pituitary hormone-producing cells is not adversely affected. From the aspect of culture cost, it is generally 1000 µM or less, preferably, 100 µM or less. In one embodiment, the concentration of DAPT in the medium is generally 0.1 to 1000 µM, preferably 1 to 100 µM (e.g., 10 µM).

In the third culture step, addition of a bone morphogenic factor signal transduction pathway activation substance to the medium is not necessary. In one embodiment, the medium used in the third culture step does not contain a bone morphogenic factor signal transduction pathway activation substance.

FGF2 used in the present invention is preferably isolated. The isolated FGF2 contained in the medium used in the third culture step is exogenously added to the medium. Therefore, in one embodiment, the present invention includes a step of exogenously adding an isolated FGF2 to the medium used in the second culture step.

In the third culture step, the cell aggregates may be treated with corticosteroids by adding corticosteroids to the medium. The treatment with corticosteroids promotes differentiation of pituitary placode and/or Rathke's pouch into pituitary hormone-producing cells other than ACTH-producing cells (i.e., GH-producing cells, PRL-producing cells, TSH-producing cells, LH-producing cells, FSH-producing cells, etc.). Examples of the corticosteroids include, but are not limited to, natural glucocorticoids such as hydrocortisone, cortisone acetate, acetic acid fludrocortisone, and the like; artificially-synthesized glucocorticoids such as dexamethasone, betamethasone, predonisolone, methylprednisolone, triamcinolone, and the like, and the like.

The concentration of corticosteroids in the medium is not particularly limited as long as it can promote differentiation of pituitary placode and/or Rathke's pouch into pituitary hormone-producing cells (excluding ACTH-producing cells). It can be appropriately determined according to the kind of the corticosteroids. For example, in the case of hydrocortisone, it is generally 100 ng/ml or more, preferably 1 µg/ml or more. The upper limit of the hydrocortisone concentration is not particularly set as long as differentiation into pituitary hormone-producing cells (excluding ACTH-producing cells) is not adversely affected. From the aspect of culture cost, it is generally 1000 µg/ml or less, preferably 100 µg/ml or less. In one embodiment, the concentration of hydrocortisone in the medium is generally 100 ng/ml to 1000 µg/ml, preferably 1 to 100 µg/ml. When dexamethasone is used as the corticosteroids, the concentration thereof in the medium can be set to about 1/25 that of hydrocortisone.

In the third culture step, the timing of adding corticosteroids to the medium is not particularly limited as long as it can promote differentiation of pituitary placode and/or Rathke's pouch into pituitary hormone-producing cells (excluding ACTH-producing cells). Corticosteroids may be added to the medium from the start of the third culture step, or corticosteroids may be added to the medium after culturing for a certain period of time in a medium free of addition of corticosteroids after the start of the third culture step. Preferably, after the start of the third culturing step, corticosteroids are added to the medium when the emergence of ACTH-producing cells is confirmed in the cell aggregates. That is, the cell aggregates are cultured in a medium free of addition of corticosteroids until the emergence of ACTH-producing cells is confirmed in the cell aggregates, and the third culture step is continued in a medium containing corticosteroids, after the emergence of ACTH-producing cells is confirmed. The emergence of ACTH-producing cells can be confirmed by immunohistological staining using an antibody against ACTH. When human pluripotent stem cells are used, the emergence of ACTH-producing cells can be generally expected after 37 days from the start of the third culture step. In one embodiment, therefore, corticosteroids are added to the medium 37 days after the start of the third culture step.

The period of treatment of cell aggregates with corticosteroids is not particularly limited as long as it can promote differentiation of pituitary placode and/or Rathke's pouch into pituitary hormone-producing cells (excluding ACTH-producing cells). In general, cell aggregates are treated with corticosteroids until promotion of differentiation into pituitary hormone-producing cells (excluding ACTH-producing cells) is confirmed in the corticosteroid treatment group as compared with a corticosteroid non-treatment group. The treatment period is generally 7 days or more, preferably 12 days or more. The upper limit of the treatment period is not particularly set and the corticosteroids may be removed from the medium at the stage when promotion of differentiation into pituitary hormone-producing cells (excluding ACTH-producing cells) is confirmed in the corticosteroid treatment group as compared with the corticosteroid non-treatment group.

The addition of corticosteroids to the medium suppresses differentiation induction of ACTH-producing cells by feedback inhibition.

The medium used in the third culture step can be prepared using a medium used for culturing mammalian cells as a basal medium, similar to the medium used in the first and the second culture step. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium-1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of pluripotent stem cells into pituitary placode and/or Rathke's pouch, and then pituitary hormone-producing cells. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into pituitary hormone-producing cells, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

The suspension culture in the third culture step is preferably performed under high oxygen partial pressure conditions. Further suspension culture of cell aggregates containing 1) hypothalamic neuroepithelial tissue, and 2) pituitary placode and/or Rathke's pouch under high oxygen partial pressure conditions allows oxygen to reach the inside of the cell aggregates and achieves maintenance culture of the cell aggregates for a long period of time, which in turn enables efficient induction of differentiation into pituitary hormone-producing cells.

The high oxygen partial pressure conditions mean oxygen partial pressure conditions above the oxygen partial pressure in air (20%). The oxygen partial pressure in the third culture step is, for example, 30 to 60%, preferably 35 to 60%, more preferably 38 to 60%.

Other culture conditions such as culture temperature, CO₂ concentration, and the like in the third culture step can be appropriately set. The culture temperature is, for example, about 30 - 40°C, preferably about 37°C. The CO₂ concentration is, for example, about 1 to 10%, preferably about 5%.

The third culture step is conducted for a period of time sufficient to induce differentiation of pituitary placode and/or Rathke's pouch into pituitary hormone-producing cells. By performing the third culture step, differentiation of pituitary placode and/or Rathke's pouch into pituitary hormone-producing cells is induced, pituitary hormone-producing cells are produced in the pituitary placode and/or Rathke's pouch, and adenohypophysis can be formed. As the pituitary hormone-producing cells induced from pituitary placode and/or Rathke's pouch, growth hormone (GH)-producing cells, prolactin (PRL)-producing cells, and adrenocorticotropic hormone (ACTH)-producing cells can be mentioned. In a preferred embodiment, the adrenocorticotropic hormone (ACTH)-producing cells secrete ACTH in response to CRH stimulation, and the ACTH secretion is feedback-suppressed by glucocorticoid. In one embodiment, differentiation of at least one type, preferably two types, more preferably three types, of pituitary hormone-producing cells selected from the group consisting of growth hormone (GH)-producing cells, prolactin (PRL)-producing cells, and adrenocorticotropic hormone (ACTH)-producing cells from pituitary placode and/or Rathke's pouch is induced, and adenohypophysis containing one type, preferably two types, more preferably three types, of pituitary hormone-producing cells selected from the group consisting of growth hormone (GH)-producing cells, prolactin (PRL)-producing cells, and adrenocorticotropic hormone (ACTH)-producing cells is formed. From pituitary placode and/or Rathke's pouch, other pituitary hormone-producing cells such as thyroid-stimulating hormone (TSH)-producing cells, follicle-stimulating hormone (FSH)-producing cells, luteinizing hormone (LH)-producing cells, melanocyte stimulation hormone (MSH)-producing cells and the like may be induced besides growth hormone (GH)-producing cells, prolactin (PRL)-producing cells, and adrenocorticotropic hormone (ACTH)-producing cells. That is, the adenohypophysis formed by the third culture step may contain other pituitary hormone-producing cells such as thyroid-stimulating hormone (TSH)-producing cells, follicle-stimulating hormone (FSH)-producing cells, luteinizing hormone (LH)-producing cells, melanocyte stimulation hormone (MSH)-producing cells and the like in addition to at least one type, preferably two types, more preferably three types, selected from the group consisting of growth hormone (GH)-producing cells, prolactin (PRL)-producing cells, and adrenocorticotropic hormone (ACTH)-producing cells. Differentiation into pituitary hormone-producing cells can be confirmed, for example, by detecting the pituitary hormone positive cell by immunohistochemistry using specific antibodies against pituitary hormone. For example, the third culture step is conducted until 10% or more, preferably 30% or more, more preferably 50% or more, of the cell aggregates in the culture contain pituitary hormone-producing cells. The culture period may vary according to the animal species of the pluripotent stem cells, and the kind of the Shh signal pathway agonist and cannot be specified. When a human pluripotent stem cell is used, the period of the third culture step is, for example, generally not less than 37 days, for example, 37 to 70 days.

By performing the third culture step, cell aggregates containing adenohypophysis can be obtained.

Through the production method of the present invention, suspension culture of aggregates may be performed in the presence or absence of feeder cells as long as differentiation of pluripotent stem cells into adenohypophysis or a progenitor tissue thereof can be induced. However, from the viewpoint of avoiding contamination with undetermined factors, it is preferable to perform suspension culture of cell aggregates in the absence of feeder cells.

In the production method of the present invention, a culture vessel used for the suspension culture of cell aggregates is not particularly limited and, for example, flask, tissue culture flask, dish, petri dish, tissue culture dish, multidish, microplate, micropore, microwell plate, multiplate, multiwall plate, chamber slide, petri dish, tube, tray, culture bag, and roller bottle can be mentioned. To enable culture under non-adhesive conditions, the culture vessel is preferably non-adhesive to cells. As the cell non-adhesive culture vessel, a culture vessel whose surface has been artificially treated to be non-adhesive to cells, a culture vessel that has not been artificially treated (for example, coating treatment with extracellular matrix, etc.) for the purpose of improving adhesion to cells, or the like can be used.

As the culture vessel to be used for the suspension culture of cell aggregates, an oxygen-permeable culture vessel may also be used. By using an oxygen-permeable culture vessel, the supply of oxygen to cell aggregates is improved, which can contribute to the long-term maintenance culture of cell aggregates.

In suspension culture of aggregates, the aggregates may be statically cultured or the aggregates may be intentionally moved by rotation culture or shaking culture, as long as the aggregates can maintain a non-adhesive state to the culture vessel. In the present invention, it is not necessary to intentionally move aggregates by rotation culture or shaking culture. That is, in one embodiment, the suspension culture in the production method of the present invention is performed by static culture. The static culture refers to a method of culturing aggregates without intentionally moving them. That is, for example, the medium is circulated as the topical medium temperature changes, and the aggregates may move due to the flow, even though the aggregates are not intentionally moved. In the present invention, such case is also included in the static culture. Static culture may be performed throughout the entire suspension culture period, or static culture may be performed only for a part of the period. In a preferred embodiment, static culture is performed throughout the entire suspension culture period. Static culture does not require equipment, is expected to cause less damage to cell aggregate, and is advantageous in that the volume of culture medium can be reduced.

### (4) Separation method of pituitary hormone-producing cells and the like using cell surface marker of the present invention

In one embodiment of the separation method of the invention, a method for separating pituitary hormone-producing cells and/or progenitor cells thereof, and hypothalamic cells (in the present specification, these cells of separation interest sometimes are referred to as "therapeutic cells") includes a step for separating CD49c-expressing cells from a cell aggregate including adenohypophysis and/or a progenitor tissue thereof and a hypothalamic neuroepithelial tissue is provided. Although CD49c can be expressed on the surface of pituitary hormone-producing cells and/or progenitor cells thereof, and hypothalamic cells, CD49c cannot be expressed on the surface of undifferentiated cells and cyst-forming cells. Therefore, pituitary hormone-producing cells and/or progenitor cells thereof, and hypothalamic cells and undifferentiated cells and cyst-forming cells can be separate by utilizing CD49c as a marker. In addition, since a cell population separated by the cell surface marker of the invention can include cells other than hypothalamic and pituitary lineages (undifferentiated cells and the like), in order to produce more therapeutically effective cell population and implant tissue (cell aggregates, cell sheets and the like), it is preferable to purify therapeutic cells.

It was confirmed that when a cell population separated by the separation method of the invention is reaggregated, several types of proliferative cells with different growth rates can be mixed. That is, it was found that based on the area of the cell population on day 8 after reaggregation of cells separated by the separation method of the invention, there is a cell population that proliferated more than 3 times after day 21, a cell population that proliferated more than 3 times after day 28, and a cell population that showed little change in area until day 36. It is assumed that the proliferative cells are the result of some undifferentiated cells or cyst-forming cells remaining and proliferating in the separated cell population. Therefore, therapeutic cells can be purified by selecting a cell population that does not proliferate even after a particular period of time from among the reaggregated cell aggregates based on the area of the cell population. In addition, typically, cells that proliferate earlier become string-like form after proliferating, and cells that proliferate later exhibit cyst hypertrophy after proliferating. Therefore, it is also possible to select a cell population with low proliferation based on the morphology of the cell population. Furthermore, since most of the proliferative cells express SSEA5, the separation method of the invention may include a step of removing SSEA5-positive (especially, SSEA5-strongly positive) cells. This step may be performed simultaneously with the step of separating CD49c-expressing cells from the cell aggregate before cell separation, or may be performed before or after this step. If performed simultaneously, for example, by staining CD49c and SSEA5 simultaneously using fluorescence-activated cell sorting and setting a gate on the CD49c-positive fraction so that a SSEA5 (high) expression population is gated out, but is not limited to this method.

In addition, the present inventors found that although EpCAM can be expressed on the surface of pituitary hormone-producing cells and/or progenitor cells thereof, it cannot be expressed on the surface of hypothalamic cells. Therefore, pituitary hormone-producing cells and/or progenitor cells thereof, and hypothalamic cells can be separated each other by using CD49c and EpCAM as markers. Thus, in another embodiment, a method for separating pituitary hormone-producing cells and/or progenitor cells thereof, which includes a step for separating cells expressing CD49c and EpCAM from a cell aggregate including adenohypophysis and/or a progenitor tissue thereof and optionally a hypothalamic neuroepithelial tissue, is provided. Typically, the step of separating cells expressing CD49c and EpCAM can be performed by separating CD49c and EpCAM copositive (double positive) cells. Alternatively, it can be performed by separating cells expressing CD49c and EpCAM from the cell population including the separated cells after separating cells expressing CD49c and EpCAM from a cell aggregate including adenohypophysis and/or a progenitor tissue thereof and optionally a hypothalamic neuroepithelial tissue.

Furthermore, as shown in the below-mentioned Examples, in a cell aggregate induced to differentiate from pluripotent stem cells, proliferative cells similar to mesenchymal stem cells may occur, and the proliferative cells may contaminate the cell population separated using CD49c as an index. Since these proliferative cells can express at least one cell surface antigen (also referred to as a negative marker of the invention) selected from the group consisting of CD90, CD29, CD44, CD58, and CD166, proliferative cells can be removed using the surface antigen as a negative marker. Therefore, the separation method of the invention may include the step of separating cells that do not express at least one of the negative markers of the invention. Such step may be performed simultaneously with a step of separating cells expressing CD49c and/or EpCAM from a cell aggregate before cell separation, or may be performed before or after this step. When carried out simultaneously, it can typically be performed by separating cells that are CD49c positive and negative for the negative marker of the invention. CD90 is particularly preferred as the negative marker of the invention.

The cell aggregate before cell separation to be used in the separation method of the present invention may be any of the cell aggregate obtained by the second culture step, and the cell aggregate obtained by the third culture step. A specific number of days after the start of differentiation induction from the aforementioned pluripotent stem cells into pituitary gland or a partial tissue thereof, or a progenitor tissue thereof is, for example, 30 days to 600 days, preferably 90 days to 500 days, more preferably 110 days to 490, further preferably 150 days to 400 days, further more preferably 200 days to 350 days. The separation method of the present invention includes the following steps.

### • Outline of separation steps of the present invention

The separation method of the invention may include (A) a step of dispersing a cell aggregate including adenohypophysis and/or a progenitor tissue thereof and optionally a hypothalamic neuroepithelial tissue to obtain a sell population of single cells before (B) a step of separating cells expressing CD49c and/or EpCAM. In the present invention, the cell to be separated may be single cells, but typically is a cell population consisting of a plurality of cells. Therefore, in the present specification, unless otherwise specified, "cell" includes "cell population." A cell population may be composed of one type of cells, or may be composed of two or more types of cells. Therefore, "pituitary hormone-producing cells and/or progenitor cells thereof, and hypothalamic cells" separated from cell aggregates by using CD49c as an index can be read as a cell population containing single or plural pituitary hormone-producing cells and/or progenitor cells thereof, and single or plural hypothalamic cells.

The step (A) of dispersing cell aggregates to obtain a population of single cells may specifically include (al) a step of dispersing cell aggregates before cell separation by an enzyme treatment (below-mentioned (4.2)). Before the step of dispersing by an enzyme treatment, (a2) a step of shredding the cell aggregates before cell separation, or physically incising the cell aggregates before cell separation may be included (below-mentioned (4.2)).

The step (A) of dispersing cell aggregates to obtain a population of single cells may optionally include (a-1) a step of treating cell aggregates before cell separation with a ROCK inhibitor. When this step is performed, it is preferably performed first in the step of obtaining a population of single cells (below-mentioned (4.1)). Before the above cell separation step (B), (C) a step of culturing the obtained cells on a culture vessel coated with laminin or a fragment thereof may optionally be performed (below-mentioned (4.3)).

After the step (a2) of dispersing by enzymatic treatment, (B) a step of separating cells expressing EpCAM (step of separating cells expressing EpCAM and cells not expressing EpCAM) is performed (below-mentioned (4.4)).

After the above-mentioned cell separation step (B), a step (D) of reaggregating the obtained cells may be optionally performed (below-mentioned (4.5)). The reaggregation step (D) may be specifically performed by (dl) seeding the obtained cells in a culture vessel and performing adhesion culture, and/or (d2) seeding the obtained cells in a culture vessel and performing suspension culture.

### (4.1) Pre-treatment step with ROCK inhibitor

In the separation method of the present invention, the obtained cell aggregates may be first pre-treated with a ROCK inhibitor. For the pre-treatment, a ROCK inhibitor is preferably added before the start of the pre-treatment in order to suppress cell death of pluripotent stem cells (particularly, human pluripotent stem cells) induced by dispersing of the cell aggregate thereafter. The ROCK inhibitor is added, for example, at least 24 hr before, at least 12 hr before, at least 6 hr before, at least 3 hr before, at least 2 hr before, at least 1 hr before, the start of the treatment. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for the treatment is a concentration that can suppress cell death of pluripotent stem cells induced by dispersing of the cell aggregate thereafter. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period. In any of the below-mentioned steps (4.2) to (4.5), as the solution to be contacted with the cells, a solution containing a ROCK inhibitor at the same concentration is preferably used.

As the medium to perform the pre-treatment, the medium used in the second culture step can be used when the cell aggregates obtained in the second culture step is used, and the medium used in the third culture step can be used when the cell aggregates obtained in the third culture step is used. When a ROCK inhibitor is already added to the above-mentioned medium at a desired concentration, the pre-treatment step is not necessary.

### (4.2) Cell aggregate dispersion step

Then, the cell aggregates that underwent the above-mentioned pre-treatment are dispersed by an enzyme treatment. Specifically, the cell aggregates that underwent the above-mentioned pre-treatment are transferred to a culture vessel (e.g., tube etc.) containing the medium (e.g., mixed medium of DMEM/F-12 medium (DMEM medium:Ham's F-12 medium=1:1), etc.) described in the second or the third culture step and washed with the same medium. The enzyme for dispersion is not particularly limited as long as it can disperse the cells. For example, EDTA; enzymes such as trypsin, collagenase (collagenase type I - VII), metalloprotease, hyaluronidase, elastase, DISPASE, deoxyribonuclease, and the like, and a mixture of these can be mentioned. A preferred enzyme is collagenase, and more preferred enzyme is collagenase type I. The conditions (temperature, time, etc.) of the enzyme treatment can be appropriately set according to the enzyme to be used and the like. To promote the enzyme treatment, a step of physically (e.g., scalpel, scissors, etc.) shredding the cell aggregate, or physically (e.g., scalpel, scissors, etc.) incising the cell aggregates may be performed before the treatment.

After the above-mentioned enzyme treatment, floating cells are recovered, and the above-mentioned enzyme treatment is performed again. The enzyme treatment can be performed using the aforementioned enzyme and the like, and preferred enzymes are EDTA; trypsin, more preferably, EDTA; trypsin and deoxyribonuclease. In addition, a commercially available product such as TrypLE (Invitrogen) or the like may also be used instead of EDTA; trypsin. The conditions (temperature, time, etc.) of the enzyme treatment can be appropriately set according to the enzyme to be used and the like. A single cell suspension can be prepared by the above-mentioned series of enzyme treatments. When preparing a single cell suspension, dead cells may be removed by a method known per se.

### (4.3) Culture step using laminin or a fragment thereof

As shown in the examples below, it was shown that by using a specific integrin complex expressed on the cells contained in a cell aggregate as an index, through this culture step, dead cells can be easily removed from the cell aggregates, and pituitary cells and hypothalamic cells with high cell viability can be purified. Specifically, cell aggregates are cultured in a culture vessel coated with laminin or a fragment thereof, which has a high affinity for the integrin α3β1 (CD49c/CD29) complex commonly expressed in pituitary cells and hypothalamic cells. Therefore, some of the cells that do not express the integrin α3β1 complex (for example, cyst-forming cells) and dead cells contained in the aggregates are present in the floating cell fraction, and the remaining cells, including pituitary cells and hypothalamic cells, are present in the adhesive cell fraction. Therefore, dead cells can be easily removed by removing the floating cell fraction. Moreover, by performing such step, cell survival rate can also be improved. Step (4.3) is preferably performed after above-mentioned (4.2) cell aggregate dispersion step.

Laminin or fragment used in step (4.3) is not particularly limited as long as it has at least a high affinity for the integrin α3β1 complex, but include laminin-111 and an E8 region-containing fragment thereof, laminin-211 and an E8 region-containing fragment thereof (e.g. iMatrix-211), laminin-121 or an E8 region-containing fragment thereof, laminin-221 or an E8 region-containing fragment thereof, laminin-332 or an E8 region-containing fragment thereof, laminin-3A11 or an E8 region-containing fragment thereof, laminin-411 or an E8 region-containing fragment thereof (e.g., iMatrix-411), laminin-421 or an E8 region-containing fragment thereof, laminin-511 or an E8 region-containing fragment thereof (e.g., iMatrix-511, iMatrix-511 silk), laminin-521 or an E8 region-containing fragment thereof, laminin-213 or an E8 region-containing fragment thereof, laminin-423 or an E8 region-containing fragment thereof, laminin-523 or an E8 region-containing fragment thereof, laminin-212/222 or an E8 region-containing fragment thereof, laminin-522 or an E8 region-containing fragment thereof and the like. Among these, laminin-511 or an E8 region-containing fragment thereof is preferred. When the step is performed, the adhesion culture itself can be performed by a method known per se, and the contents described in the aforementioned second culture step and third culture step (e.g., medium composition, etc.) may also be used as appropriate.

The specific period for culturing the cell aggregates in a culture vessel coated with laminin or a fragment thereof is, for example, 30 minutes to 5 hours, preferably 1 hour to 3 hours.

After the adhesive culture step, the adhesive cells can be detached and collected by performing a dissociation enzyme treatment. The dissociating enzyme is not particularly limited as long as it can detach and collect the desired cells, but it is desirable to use an enzyme that does not contain animal-derived components, and preferred enzymes include commercial products such as TrypLE (Invitrogen). Conditions (temperature, time, etc.) for the dissociation enzyme treatment can be appropriately set depending on the enzyme used.

### (4.4) Separation step of CD49c and/or EpCAM-positive cell

As a method for separating the desired cells that express CD49c and/or EpCAM from the above-mentioned cell population contained in the single cell suspension prepared above, methods using flow cytometry and mass cytometry, magnetic cell separation methods, and the like can be mentioned. These methods can be carried out using a method known per se. For example, cell that expresses CD49c and/or EpCAM can be separated by a method including a step of contacting the cells and a substance (e.g., antibody, etc.) that specifically binds to CD49c or EpCAM molecule. The above-mentioned substance includes those with detectable labels (e.g., GFP, PE) attached to themselves and those without labels attached to themselves. When the above-mentioned substance does not have a label attached thereto, the separation can be achieved by further using a substance with a detectable label attached thereto that directly or indirectly recognizes the substance. For example, when the aforementioned substance is an antibody, then a fluorescent dye, a metal isotope, or a bead (e.g., magnetic bead) is directly or indirectly carried on the antibody to label a cell surface marker. The cells can be separated based on the label. The antibody used at this time may be one kind of antibody, or two or more kinds of antibodies.

When separating CD49c-positive cells labeled with a fluorescent dye from CD49c-negative cells using flow cytometry, due to the influence of cell autofluorescence (fluorescence emitted by the cells themselves regardless of CD49c expression), CD49c fluorescence staining alone may have poor population separation of CD49c-positive cells and CD49c-negative cells based on fluorescence intensity. In such a case, as shown in the Examples below, the fluorescence intensity range of CD49c-negative cells is determined by simultaneously labeling undifferentiated cells included in CD49c-negative cells using another fluorescent dye. Cells whose fluorescence intensity is stronger than the fluorescence intensity range of CD49c-negative cells determined in this manner are treated as CD49c-positive cells. A method for labeling undifferentiated cells with a fluorescent dye includes a method for labeling with a fluorescent dye via an antibody against SSEA5, which is a surface antigen of undifferentiated cells.

In the present specification, "positive" includes cases of high expression (also referred to as "strongly positive"). High expression may be expressed as High or Bright. For example, cells that highly express SSEA5 may be referred to as SSEA5^{High} cells or SSEA5^{Bright} cells.

High expression can be determined based on a chart obtained by a flow cytometry method. The position appearing in the chart may vary depending on the voltage setting of the instrument, sensitivity setting, antibody clone used, staining conditions, dye used, etc. If a person skilled in the art, a cell population recognized as a group in the obtained chart is considered. Lines can be drawn appropriately so as not to be cut.

### (4.5) Reaggregation culture step of separated cell population

The separated cell population may be seeded in a culture vessel and subjected to adhesion culture to give, for example, a cell sheet, or may be seeded in a culture vessel and subjected to suspension culture to allow for reaggregation to form a cell aggregate. The cell population may be maintenance cultured as it is until use, or may be further induced to differentiate into desired cells according to the differentiation state of the separated cells. When the maintenance culture or further differentiation induction is performed, the method described in the aforementioned third culture step may be performed as it is, or where necessary, it may be performed by appropriately modifying by a method known per se. When the adhesion culture is performed, the adhesion culture itself can be performed by a method known per se, and the contents described in the aforementioned third culture step (e.g., medium composition, etc.) may also be used as appropriate. During the adhesion culture, it is preferable to coat the culture vessel with an extracellular matrix or the like (e.g., laminin, collagen, etc.).

### (5) Use of separated pituitary hormone-producing cells, progenitor cells thereof and tissues containing thereof

Pituitary hormone-producing cells and progenitor cells thereof obtained by the separation method or production method of the invention, and cell aggregates and cell sheets containing the cells (the cell aggregates and cell sheets are referred to as "pituitary tissue") can be used for transplant medical treatment (in other words, as a transplant therapeutic agent). The pituitary tissue may include hypothalamic cells. For example, the pituitary hormone-producing cells, progenitor cells thereof and/or pituitary tissue obtained by the separation method or the production method of the present invention can be used as a therapeutic drug for diseases based on disorders of the adenohypophysis (anterior or intermediate lobe, preferably anterior lobe), or for supplementing the corresponding damaged portion in the damaged states of the adenohypophysis (anterior or intermediate lobe, preferably the anterior lobe). By transplanting the pituitary hormone-producing cells, progenitor cells thereof and/or pituitary tissue obtained by the separation method or the production method of the present invention to patients with diseases based on disorders of the adenohypophysis or in the damaged states of the adenohypophysis, the diseases based on disorders of the adenohypophysis and the damaged states of the adenohypophysis can be treated. The transplantation site is not particularly limited as long as the transplanted pituitary hormone-producing cells, progenitor cells thereof and/or pituitary tissue can function as a substitute for the damaged adenohypophysis and, for example, under the renal capsule and the like can be mentioned. As the diseases based on disorders of the adenohypophysis, panhypopituitarism, pituitary dwarfism, adrenocortical insufficiency, partial hypopituitarism, pituitary anterior hormone isolated deficiency, and the like can be mentioned. As the damaged states of the adenohypophysis, conditions of adenohypophyses of patients after adenohypophysectomy, conditions of adenohypophyses of patients after radiation to pituitary tumors, and trauma of adenohypophyses can be mentioned.

In transplantation therapy, rejection due to differences in histocompatibility antigens often poses a problem. Such problem can be overcome by using pluripotent stem cells (e.g., iPS cells) established from the somatic cells of recipients of transplantation. That is, in a preferred embodiment, pluripotent stem cells (e.g., iPS cells) established from the somatic cells of recipients are used as pluripotent stem cells in the method of the present invention to produce adenohypophysis or a progenitor tissue thereof, or a pituitary hormone-producing cell, which is immunologically self for the recipient, and this is transplanted into the recipient. iPS cells established from somatic cells whose HLA genotype is the same or substantially the same as that of the individual to which the cells are to be transplanted are also preferably used. The term "substantially the same" herein means that the HLA genotype is matching to an extent at which the immune reaction against the transplanted cells can be suppressed with an immunosuppressive agent. For example, the somatic cells have matched HLA types at 3 loci HLA-A, HLA-B, and HLA-DR, or at the 4 loci further including HLA-C. Furthermore, in order to suppress rejection, cells whose HLA genes have been modified by genetic engineering techniques may be used. Such methods include a method of preparing HLA pseudo homozygotes by selectively removing the HLA gene portion on one side of the chromosome in iPS cells derived from common HLA heterozygote donors, a method of destroying HLA-A and HLA-B genes while maintaining HLA-C on one side of the chromosome to suppress the reaction of NK cells and the like.

Furthermore, the pituitary hormone-producing cells, progenitor cells thereof, and pituitary tissue obtained by the method of the present invention can be used for screening and evaluation of drugs. Specifically, they can be applied to, for example, screening for substances that suppress or promote the production of pituitary hormones, side effects and toxicity tests of pharmaceutical products, and the like. The above-mentioned screening, test, and the like may contain, for example, a step of culturing a cell population or pituitary tissue containing pituitary hormone-producing cells and/or progenitor cells thereof in the presence or absence (negative control) of a test substance, a step of comparing the production amount of the desired hormone in a cell population or pituitary tissue treated with a test substance with that of a negative control, and a step of selecting, as a candidate substance, a test substance that suppresses or promotes the production of the pituitary hormone.

### (6) Production method of pituitary hormone-producing cells, progenitor cells thereof and/or pituitary tissue of the present invention

The present invention provides a method for producing pituitary hormone-producing cells, progenitor cells thereof and/or pituitary tissue, including a step of separating the cells that express CD49c from cell aggregates containing adenohypophysis and/or a progenitor tissue thereof, and hypothalamic neuroepithelial tissue. CD49c can be expressed on the surface of pituitary hormone-producing cells and progenitor cells thereof, and hypothalamic cells, but cannot be expressed on the surface of undifferentiated cells and cyst-forming cells. Therefore, pituitary hormone-producing cells and/or progenitor cells thereof, and hypothalamic cells and undifferentiated cells and cyst-forming cells can be separate by utilizing CD49c as a marker. Using the separation step, a cell population containing pituitary hormone-producing cells, progenitor cells thereof and/or pituitary tissue, or pituitary hormone-producing cells, progenitor cells thereof and/or pituitary cell in a high purity (e.g., 80% or more, preferably 85% or more, more preferably 90%, further preferably 95% or more, further more preferably 99% or more, most preferably 100% of the cell population) can be produced.

As mentioned above, EpCAM can be expressed on the surface of the pituitary hormone-producing cells and progenitor cells thereof, but cannot be expressed on the surface of the hypothalamic cells. Therefore, pituitary hormone-producing cells and progenitor cells thereof, and hypothalamic cells can be separated by CD49c and EpCAM as markers. Therefore, in another embodiment of the present invention, a method for separating pituitary hormone-producing cells and/or progenitor cells thereof, which includes a step of separating cells expressing CD49c and EpCAM from a cell aggregate including adenohypophysis and/or a progenitor tissue thereof and optionally a hypothalamic neuroepithelial tissue. The step of separating cells expressing CD49c and EpCAM can typically be performed by separating CD49c and EpCAM co-positive cells. Alternatively, it may be carried out by separating cells expressing CD49c or EpCAM from a cell aggregate including adenohypophysis and/or a progenitor tissue thereof and optionally a hypothalamic neuroepithelial tissue, and then separating cells expressing EpCAM or CD49c from a cell population containing the separated cells. Using the separation step, a cell population containing pituitary hormone-producing cells, progenitor cells thereof and/or pituitary tissue, or pituitary hormone-producing cells and/or progenitor cells thereof in a high purity (e.g., 80% or more, preferably 85% or more, more preferably 90%, further preferably 95% or more, further more preferably 99% or more, most preferably 100% of the cell population) can be produced.

Furthermore, as mentioned above, proliferative cells similar to mesenchymal stem cells occur in cell aggregates induced to differentiate from pluripotent stem cells, and the proliferative cells may contaminate the cell population separated using CD49c as an index. These proliferative cells can be removed using a negative marker of the present invention as an index. Therefore, the production method of the invention can include a step of separating cells that do not express at least one of the negative markers of the present invention. Such step may be performed simultaneously with a step of separating cells expressing CD49c from a cell aggregate including adenohypophysis and/or a progenitor tissue thereof, or may be performed before or after this step. When carried out simultaneously, it can typically be carried out by separating cells that are CD49c positive and negative for the negative marker of the invention. CD90 is particularly preferred as the negative marker of the invention.

As mentioned above, in the production method of the invention, cell populations and implant tissues (cell aggregates, cell sheets, etc.) with a higher therapeutic effect can also be produced by selecting cell populations with low proliferation from the reaggregated cell aggregates based on the area of the cell population. In addition, typically, cells that proliferate earlier become string-like form after proliferating, and cells that proliferate later exhibit cyst hypertrophy after proliferating. Therefore, it is also possible to select a cell population with low proliferation based on the morphology of the cell population. Furthermore, since most of the proliferative cells express SSEA5, the production method of the invention may include a step of removing SSEA5-positive (particularly, strongly SSEA5-positive) cells.

In the step of separating the cells that express CD49c and/or EpCAM, and/or the cells that express the negative marker of the present invention and/or SSEA5 from cell aggregates before cell separation, contained in the production method of the present invention, all of the contents described in the aforementioned "Outline of separation step of the present invention" may be used. In addition, all of the contents of the aforementioned (4.1) to (4.5) can be used for each step.

The pituitary hormone-producing cells and/or progenitor cells thereof, and the like produced by the method of the present invention may be, as described in the above-mentioned (4.1), maintenance cultured as they are until use, or may be further induced to differentiate into desired cells according to the differentiation state of the separated cells, by appropriately culturing the cells (e.g., the third culture step). Specifically, for example, the pituitary hormone-producing cells, progenitor cells thereof, and/or pituitary tissue produced by the method of the present invention may be directly transplanted to a subject in need of the treatment and the like with the cell population or tissue, or may be further processed by a method known per se to achieve higher purity (purified) and then transplanted to the subject. The purified cells may be seeded in a culture vessel and adhesion cultured as described in the above-mentioned (4.5) and, for example, may be shaped like a cell sheet, or seeded in a culture vessel and cultured in suspension to cause reaggregation. A cell sheet or cell aggregate obtained through the adhesion culture or suspension culture may be transplanted to the above-mentioned subject. The adhesion culture or suspension culture may be performed for a short period of time (e.g., 3 to 6 days) or for a long period of time (e.g., 7 to 30 days). In the production method of the present invention, all of the contents relating to the aforementioned "(1) Pluripotent stem cell" to "(5) Use of separated pituitary hormone-producing cells, progenitor cells thereof and tissues containing thereof' may be used.

### (7) Reagent for separation of pituitary hormone-producing cells and/or progenitor cells thereof, and hypothalamic cells

The present invention also provides a reagent for separation of pituitary hormone-producing cells and/or progenitor cells thereof, and hypothalamic cell containing an antibody against CD49c (hereinafter, sometimes referred to as "reagent of the invention").

Additionally, the reagent of the invention may further contain an antibody against EpCAM. By containing an antibody against EpCAM, the reagent of the invention can also be used as a reagent for separation of pituitary hormone-producing cells and/or progenitor cells thereof. When the reagent of the invention includes more than one type of antibody, the reagent can be provided as a reagent kit containing each antibody in a separate reagent. Depending on the separation means used in the method of the invention, the antibodies contained in the reagent of the invention can be provided, for example, in a form bound to fluorescent dyes, metal isotopes or beads (e.g., magnetic beads). Furthermore, the reagent of the invention may further contain antibodies against each of one or more surface antigens selected from the group consisting of CD90, CD29, CD44, CD58, CD166, and SSEA5.

While the present invention is explained in more detail in the following by referring to Examples, the Examples show mere exemplification of the present invention and do not limit the scope of the present invention in any manner.

### [EXAMPLES]

### Example 1: Screening for cell surface antigen

Human iPS cells (201B7 strain) were induced to differentiate into cell aggregates containing adenohypophysis or a progenitor tissue thereof, by the known method and described in WO 2016/013669 or Cell Reports, 30, 18-24, January 7, 2020. The cell aggregates on day 47 from the differentiation induction of the human iPS cells (201B7 strain) were dispersed by treating with Accumax (Innovative Cell Technologies, #AM105) at 37°C for 5 to 10 min to prepare a single cell suspension. The cells were stained with human surface antigen PE-labeled antibody panel (LEGENDScreen Human PE Kit, #700007) manufactured by BioLegend, and subjected to fixation and permeabilization treatment using IntraStain (Dako, #K2311). Successively, intracellular Cytokeratin staining with FITC-labeled anti-Cytokeratin antibody (Miltenyi, #130-080-101), nuclear staining with Hoechst 33342, and cytoplasm staining with HCS CellMask Deep Red Stain (Invitrogen, #H32721) were performed. The images of the cells after staining were obtained and analyzed by an imaging analyzer (Opera Phenix) manufactured by PerkinElmer, and surface antigens with a high coexistence rate in cytokeratin-positive cells were searched for. As a result, it was suggested that CD49c can identify pituitary progenitor cells.

### Example 2: Immunocytochemistry

Cell aggregates cultured for 152 days or more after the start of differentiation induction and cell aggregates reaggregated after iMatrix-511 adhesion treatment or MACS purification and cultured for 7 days or more were used. The cell aggregates were fixed with 4% paraformaldehyde solution, and sucrose replacement was performed by stepwise immersion in 10, 20, and 30% sucrose solutions. Successively, the cell aggregates were embedded with an OCT compound, and cryosections with a thickness of 4 to 10 µm were produced with a cryostat and attached to anti-peeling coated slide glass (PLATINUM PRO; Matsunami Glass). The sections were washed with PBS, and blocked with a blocking solution (5% normal donkey serum + 0.1% TritonX-100 in PBS) for 30 to 60 min at room temperature. Then, they were reacted overnight at 4°C with a primary antibody diluted with a blocking solution. Successively, they were reacted at room temperature for 1 hr with a fluorescence-labeled secondary antibody and DAPI (nuclear staining agent). The sections after staining were mounted with Fluoromount (Diagnostic BioSystems, #K024) and subjected to fluorescence observation with a confocal laser microscope LSM-710 (Zeiss).

### Example 3: Separation of floating cell fraction and adhesive cell fraction by iMatrix-511 adhesion treatment

Cell aggregates 110 to 490 days after initiation of differentiation induction were enzymatically dispersed and used for iMatrix-511 adhesion treatment. The procedure is shown below.

### (1) Pre-treatment with Y27632

In order to suppress cell death due to dispersion treatment, Y27632 (Wako, #034-24024) was added to the medium at a final concentration of 20 µM one hour or more before the start of the work to pre-treat cell aggregates. In subsequent operations, 20 µM Y27632 was added to all solutions (except PBS) to which the cells were exposed.

### (2) Dispersion of cell aggregates

In order to promote dispersion by enzyme treatment, the cell aggregates were shredded or incised with a scalpel. Cell aggregates were then transferred to a 50 ml tube and washed with DMEM/F12 (Wako, #042-30555). Then 1-3 ml of collagenase solution was added and the mixture was rotated or shaken (140-150 rpm) at 37°C for 40 min. The composition of the collagenase solution was the above-mentioned DMEM/F12 supplemented with 0.2%-0.3% collagenase type I (Wako, #031-17601) and 0.1% BSA (Sigma, #A9418).

After the completion of collagenase treatment, the supernatant containing floating cells was transferred to a new 15 ml tube, and the remaining cell aggregates were washed with PBS (washing liquid was also collected in the same tube as the supernatant). The 15 ml tube in which the supernatant was collected was centrifuged at 4°C, 1000 rpm for 5 min to pelletize floating cells. 1 ml of 10x TrypLE Select (Gibco, #25200072) + 0.2 mg/ml DNase I (Roche, #11284932001) was added to the cell aggregates in the 50 ml tube, gyratory shaken (140-150 rpm) at 37°C for 10 min, combined with the pellets in the 15 ml tube, and the cell aggregates were loosened by pipetting about 20 times with a P1000 micropipette. After suspending in 10 ml of gfCDM + 20% KSR (medium for differentiation and maintenance of cell aggregates) to neutralize 10x TrypLE Select, the cells were centrifuged at 4°C, 1000 rpm for 5 min. 1 ml of gfCDM + 20% KSR + 10 µg/ml DNase I was added to the cell pellets after centrifugation, vigorously pipetted about 30 times with a P1000 micropipette to disperse the cell aggregates, and aggregates were removed through a 70 µm cell strainer. A single cell suspension was prepared by the above operation.

### (3) iMatrix-511 adhesion treatment

The dispersed cells were plated on a 10 cm dish coated with iMatrix-511 and cultured at 37°C for 2 hours. After culturing, the floating cells were collected together with the culture medium to obtain a floating cell fraction. Then, PBS was added to the dish to wash the surface of adherent cells. After removing PBS, 1 ml of 10x TrypLE Select + 0.2 mg/ml DNase I was added, and the mixture was incubated at 37°C. for 5 minutes. 2 ml of gfCDM + 20%KSR was added and the cells were detached by pipetting with a P1000 micropipette, and the cell suspension was collected into a conical tube. At this point, if there were any adherent cells remaining in the dish that had not been detached, the above detachment step was repeated again to collect all the adherent cells. An adherent cell fraction was obtained by the above detachment process.

### Example 4: Purification of CD49c-positive cell by magnetic cell separation method (Magnetic-Activated Cell Sorting; MACS)

Cell aggregates 110 to 490 days after initiation of differentiation induction were enzymatically dispersed and used for MACS. The procedure is shown below.

### (1) Pre-treatment with Y27632

In order to suppress cell death due to dispersion treatment, Y27632 (Wako, #034-24024) was added to the medium at a final concentration of 20 µM one hour or more before the start of the work to pre-treat cell aggregates. In subsequent operations, 20 µM Y27632 was added to all solutions (except PBS) to which the cells were exposed.

### (2) Dispersion of cell aggregates

In order to promote dispersion by enzyme treatment, the cell aggregates were shredded or incised with a scalpel. Cell aggregates were then transferred to a 50 ml tube and washed with DMEM/F12 (Wako, #042-30555). Then 1-3 ml of collagenase solution was added and the mixture was rotated or shaken (140-150 rpm) at 37°C for 40 min. The composition of the collagenase solution was the above-mentioned DMEM/F12 supplemented with 0.2%-0.3% collagenase type I (Wako, #031-17601) and 0.1% BSA (Sigma, #A9418).

After the completion of collagenase treatment, the supernatant containing floating cells was transferred to a new 15 ml tube, and the remaining cell aggregates were washed with PBS (washing liquid was also collected in the same tube as the supernatant). The 15 ml tube in which the supernatant was collected was centrifuged at 4°C, 1000 rpm for 5 min to pelletize floating cells. 1 ml of 10x TrypLE Select (Gibco, #25200072) + 0.2 mg/ml DNase I (Roche, #11284932001) was added to the cell aggregates in the 50 ml tube, incubated at 37°C for 5-10 min, combined with the pellets in the 15 ml tube, and the cell aggregates were loosened by pipetting about 20 times with a P1000 micropipette. After suspending in 10 ml of gfCDM + 20% KSR (medium for differentiation and maintenance of cell aggregates) to neutralize 10x TrypLE Select, the cells were centrifuged at 4°C, 1000 rpm for 5 min. 1 ml of gfCDM + 20% KSR + 10 µg/ml DNase I was added to the cell pellets after centrifugation, vigorously pipetted about 30 times with a P1000 micropipette to disperse the cell aggregates, and aggregates were removed through a 70 µm cell strainer. A single cell suspension was prepared by the above operation.

### (3) iMatrix-511 adhesion treatment

The dispersed cells were plated on a 10 cm dish coated with iMatrix-511 and cultured at 37°C for 2 hours. After culturing, the floating cells were discarded together with the culture medium, and PBS was added to the dish to wash the surface of the adherent cells. After removing PBS, 1 ml of 10× TrypLE Select + 0.2 mg/ml DNase I was added and incubated at 37°C for 5 minutes. 2 ml of gfCDM + 20%KSR was added and the cells were detached by pipetting with a P1000 micropipette, and the cell suspension was collected into a conical tube. At this point, if there were any adherent cells remaining in the dish that had not been detached, the above detachment step was repeated again to collect all the adherent cells. An adherent cell fraction was obtained by the above detachment process.

### (4) Separation of CD49c-positive cells by MACS

The collected cells by iMatrix-511 adhesion treatment were suspended in MACS buffer (PBS + 0.5% BSA + 2 mM EDTA) and stained with PE-labeled anti-CD49c antibody (BioLegend, #343803). CD49c-positive cells were successively labeled with magnetic beads by treating with anti-PE-microbeads (Miltenyi, #130-105-639). Then the cells were passed through a magnetic column (LS column; Miltenyi, #130-042-401) to separate and collect magnetic bead-labeled cells (CD49c-positive cells) and unlabeled cells (CD49c-negative cells).

### Example 5: Purification of CD49c-positive cells or CD49c-positive/EpCAM-positive cells by Fluorescence-Activated Cell Sorting (FACS)

Cell aggregates 110 to 490 days after initiation of differentiation induction were enzymatically dispersed and used for FACS. The procedure is shown below.

### (1) Pre-treatment with Y27632

In order to suppress cell death due to dispersion treatment, Y27632 (Wako, #034-24024) was added to the medium at a final concentration of 20 µM one hour or more before the start of the work to pre-treat cell aggregates. In subsequent operations, 20 µM Y27632 was added to all solutions (except PBS) to which the cells were exposed.

### (2) Dispersion of cell aggregates

In order to promote dispersion by enzyme treatment, the cell aggregates were shredded or incised with a scalpel. Cell aggregates were then transferred to a 50 ml tube and washed with DMEM/F12 (Wako, #042-30555). Then 1-3 ml of collagenase solution was added and the mixture was rotated or shaken (140-150 rpm) at 37°C for 40 min. The composition of the collagenase solution was the above-mentioned DMEM/F12 supplemented with 0.2%-0.3% collagenase type I (Wako, #031-17601) and 0.1% BSA (Sigma, #A9418).

After the completion of collagenase treatment, the supernatant containing floating cells was transferred to a new 15 ml tube, and the remaining cell aggregates were washed with PBS (washing liquid was also collected in the same tube as the supernatant). The 15 ml tube in which the supernatant was collected was centrifuged at 4°C, 1000 rpm for 5 min to pelletize floating cells. 1 ml of 10x TrypLE Select (Gibco, #25200072) + 0.2 mg/ml DNase I (Roche, #11284932001) was added to the cell aggregates in the 50 ml tube, incubated at 37°C for 5-10 min, combined with the pellets in the 15 ml tube, and the cell aggregates were loosened by pipetting about 20 times with a P1000 micropipette. After suspending in 10 ml of gfCDM + 20% KSR (medium for differentiation and maintenance of cell aggregates) to neutralize 10x TrypLE Select, the cells were centrifuged at 4°C, 1000 rpm for 5 min. 1 ml of gfCDM + 20% KSR + 10 µg/ml DNase I was added to the cell pellets after centrifugation, vigorously pipetted about 30 times with a P1000 micropipette to disperse the cell aggregates, and aggregates were removed through a 70 µm cell strainer. A single cell suspension was prepared by the above operation.

### (3) iMatrix-511 adhesion treatment

The dispersed cells were plated on a 10 cm dish coated with iMatrix-511 and cultured at 37°C for 2 hours. After culturing, the floating cells were discarded together with the culture medium, and PBS was added to the dish to wash the surface of the adherent cells. After removing PBS, 1 ml of 10× TrypLE Select + 0.2 mg/ml DNase I was added and incubated at 37°C for 5 minutes. 2 ml of gfCDM + 20%KSR was added and the cells were detached by pipetting with a P1000 micropipette, and the cell suspension was collected into a conical tube. At this point, if there were any adherent cells remaining in the dish that had not been detached, the above detachment step was repeated again to collect all the adherent cells. An adherent cell fraction was obtained by the above detachment process.

### (4) Separation of CD49c-positive cells or CD49c-positive/EpCAM-positive cells by FACS

Cells collected by iMatrix-511 adhesion treatment were suspended in FACS buffer (PBS + 0.5% BSA+ 2 mM EDTA). When separating CD49c-positive cells, CD49c-positive cells and CD49c-negative cells were separated and collected by using a Beckman Coulter cell sorter (MoFlo Astrios) after staining the cells with APC-labeled anti-CD49c antibody (BioLegend, #343808) and PE-labeled anti-SSEA5 antibody (BioLegend, #355203). When separating CD49c-positive/EpCAM-positive cells, CD49c-positive/EpCAM-positive cells and CD49-positive/EpCAM-negative cells were separated and collected by using the above cell sorter after staining the cells with APC-labeled anti-CD49c antibody and PE-labeled anti-EpCAM antibody (Miltenyi, #130-098-115).

### Example 6: Reaggregation culture of purified cells

Each purified cell population (iMatrix-511-adhesive cells, CD49c-positive cells, CD49c-negative cells, CD49c-positive/EpCAM-positive cells, CD49c-positive/EpCAM-negative cells) was seeded on a low-adhesion V-bottom 96-well plate (PrimeSurface plate 96V; Sumitomo Bakelite Co., Ltd., #MS-9096V) and allowed to reaggregate. 15,000 cells in 200 µl medium (gfCDM + 20% KSR + 30 µM Y27632) were seeded per well. Thereafter, half of the medium was replaced every 3 days with a medium not containing Y27632.

### Example 7: Quantification of each index

### (1) Percentage of EpCAM-positive cells and CD49c-positive cells in Rx::Venus-positive cells

After dispersing the cell aggregates to prepare a single cell suspension and staining with APC-labeled anti-EpCAM antibody (Miltenyi, #130-113-822) orAPC-labeled anti-CD49c antibody (BioLegend, #343808), the percentage of EpCAM-positive cells and CD49c-positive cells in the Rx::Venus-positive cell fraction were measured by flow cytometry (experimental method for quantitative analysis based on cell fluorescence intensity).

### (2) Percentage of cells present in the floating cell fraction and cells present in the adherent cell fraction in the iMatrix-511 adhesion treatment process

A single cell suspension was prepared by dispersing the cell aggregates and cultured in an incubator coated with iMatrix-511. Adherent cells and floating cells were collected and counted using a hemocytometer.

### (3) Percentage of CD49c-positive cells and Rx::Venus-positive cells in floating cell fraction and adherent cell fraction in iMatrix-511 adhesion treatment process

After dispersing the cell aggregates to prepare a single cell suspension and culturing it in a culture vessel coated with iMatrix-511, staining with PE-labeled anti-CD49c antibody (BioLegend, #343803), and images were acquired using a fluorescence microscope (Leica DMI6000B). CD49c-positive cells and Rx::Venus-positive cells were counted using the Cell counter tool of the image analysis software Image J, and the percentage of each in the total cells was calculated.

### (4) Cell viability in floating cell fraction and adherent cell fraction in iMatrix-511 adhesion treatment process

After dispersing the cell aggregates to prepare a single cell suspension and culturing it in a culture vessel coated with iMatrix-511, staining with APC-labeled Annexin V (BioLegend, #640919) and Propidium iodide (PI) was performed. The percentage of viable cells (Annexin V negative/PI negative cells) was calculated by flow cytometry.

### (5) Quantification of positive rate in surface marker search results for mesenchymal stem cell-like proliferative cells using detached tissues

Detached tissues derived from hiPSC samples were collected, treated with 10x TrypLE select to disperse them, fluorescently stained for surface markers of stem cell lineage, and the positive rate was calculated using a flow cytometer.

### (6) Cell viability by iMatrix-511 adhesion treatment

After dispersing hESC-derived cell aggregates (Day 448) induced by the feeder-free differentiation method, 15,000 cells each collected by Method A (collecting live cells with MACS dead cell removal kit) and Method B (collecting iMatrix-511 adherent cell fraction) were reaggregated and observed 3 days later. Using the image analysis software ImageJ, the area of the aggregates was measured on a two-dimensional image with n = 6-8.

### (7) Reaggregate size after iMatrix-511 adhesion process

CD49c- and/or EpCAM-positive cells were separated and collected by iMatrix-511 adhesion treatment, and then reaggregated and observed on Day 7 and Day 28. Using the image analysis software ImageJ, the area of the aggregates was measured on a two-dimensional image with n = 9.

### (8) Reaggregate size after separation of CD49c-positive cells and CD49c-negative cells by FACS after iMatrix-511 adhesion treatment

After iMatrix-511 adhesion treatment, CD49c-positive cells were separated and collected using FACS, and then reaggregated and observed on Day 8 and Day 36. The area of the aggregate was measured on a two-dimensional image using image analysis software ImageJ.

### Example 8: ACTH secretion test (CRH stimulation test)

Cell aggregates that were reaggregated after MACS or FACS purification and cultured for 8 days or more were used. One or five cell aggregates were incubated at a predetermined temperature and for a predetermined time using a predetermined medium, etc., and then the medium and the like were collected. ACTH concentration in the collected medium etc., was measured by the ECLIA method used in clinical testing. In addition, when confirming the influence of secretion suppressing factors and CRH receptor inhibitors, pretreatment was performed for a predetermined period of time with a maintenance medium containing secretion suppressing factors and CRH receptor inhibitors before secretion measurement.

### [INDUSTRIAL APPLICABILITY]

The separation method and the production method of the present invention can be used to efficiently separate and purify functional pituitary hormone-producing cells and/or progenitor cells thereof from differentiated tissues derived from human pluripotent stem cells, by utilizing CD49c as a marker. The separated and purified pituitary hormone-producing cells and the like exhibit superior pituitary hormone secreting ability by physiological stimulation of pituitary hormone secretion, and can be used for the treatment of the diseases relating to pituitary gland, using the cells.

This application is based on a patent application No. 2021-158406 filed in Japan (filing date: September 28, 2021), the contents of which are incorporated in full herein.

## Claims

1. A method for separating pituitary hormone-producing cells and/or progenitor cells thereof, and hypothalamic cells, comprising a step of separating cells that express CD49c from a cell aggregate comprising adenohypophysis and/or a progenitor tissue thereof, and a hypothalamic neuroepithelial tissue.

2. A method for separating pituitary hormone-producing cells and/or progenitor cells thereof, comprising a step of separating cells that express CD49c and EpCAM from a cell aggregate comprising adenohypophysis and/or a precursor tissue thereof.

3. The method according to claim 1 or 2, wherein the step of separating the cells that express CD49c, or the cells that express CD49c and EpCAM comprises the following steps:
(1) a first step of dispersing the cell aggregate into a cell population,
(2) a second step of culturing the cell population obtained in the first step on a culture vessel coated with laminin or a fragment thereof, and
(3) a third step of separating cells that express CD49c or cells that express CD49c and EpCAM from the cell population obtained in the second step.

4. The method according to any one of claims 1 to 3, comprising a step of separating cells negative for at least one cell surface antigen selected from the group consisting of CD90, CD29, CD44, CD58 and CD166.

5. The method according to any one of claims 1 to 4, wherein the cell aggregate is a cell aggregate obtained by inducing differentiation of pluripotent stem cells.

6. The method according to any one of claims 1 to 5, wherein the progenitor tissue is pituitary placode and/or Rathke's pouch.

7. A method for producing pituitary hormone-producing cells and/or progenitor cells thereof, and hypothalamic cells, comprising a step of separating cells that express CD49c from a cell aggregate comprising adenohypophysis and/or a progenitor tissue thereof, and a hypothalamic neuroepithelial tissue.

8. A method for producing pituitary hormone-producing cells and/or progenitor cells thereof, comprising a step of separating cells that express CD49c and EpCAM from a cell aggregate comprising adenohypophysis and/or a precursor tissue thereof.

9. A method for producing a cell aggregate of a cell sheet comprising pituitary hormone-producing cells and/or progenitor cells thereof, and hypothalamic cells, comprising the following steps:
(A) a step of separating cells that express CD49c from a cell aggregate comprising adenohypophysis and/or a progenitor tissue thereof, and a hypothalamic neuroepithelial tissue,
(B) a step of reaggregating a population obtained in the step of (A).

10. A method for producing a cell aggregate or a cell sheet comprising pituitary hormone-producing cells and/or progenitor cells thereof, comprising the following steps:
(a) a step of separating cells that express CD49c from a cell aggregate comprising adenohypophysis and/or a progenitor tissue thereof,
(b) a step of reaggregating a population obtained in the step of (a).

11. The method according to any one of claims 7 to 10, wherein the step of separating the cells that express CD49c, or the cells that express CD49c and EpCAM comprises the following steps:
(1) a first step of dispersing the cell aggregate into a cell population,
(2) a second step of culturing the cell population obtained in the first step on a culture vessel coated with laminin or a fragment thereof, and
(3) a third step of separating cells that express CD49c or cells that express CD49c and EpCAM from the cell population obtained in the second step.

12. The method according to any one of claims 7 to 11, comprising a step of separating cells negative for at least one cell surface antigen selected from the group consisting of CD90, CD29, CD44, CD58 and CD166.

13. The method according to any one of claims 7-12, wherein the pituitary hormone-producing cell is at least one type selected from the group consisting of growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, and luteinizing hormone (LH)-producing cells.

14. A cell aggregate or a cell sheet produced by the method according to any one of claims 9 to 13.

15. A transplant therapeutic agent comprising a cell aggregate or a cell sheet produced by the method according to any one of claims 9 to 13.

16. The transplant therapeutic agent according to claim 15 for treating a disease based on a disorder of adenohypophysis and/or a damaged state of adenohypophysis.

17. The transplant therapeutic agent according to claim 16, wherein the disease based on the disorder of adenohypophysis is selected from the group consisting of panhypopituitarism, pituitary dwarfism, adrenocortical insufficiency, partial hypopituitarism and pituitary anterior hormone isolated deficiency.

18. A therapeutic agent for a disease based on a disorder of adenohypophysis or a damaged state of adenohypophysis comprising a cell aggregate or a cell sheet produced by the method according to any one of claims 9 to 13.

19. A method for treating a disease based on a disorder of adenohypophysis or a damaged state of adenohypophysis, comprising transplanting an effective amount of a cell aggregate or a cell sheet produced by the method according to any one of claims 9 to 13 in a subject in need thereof.

20. A cell aggregate or a cell sheet according to any one of claims 9 to 13 for use in treating a disease based on a disorder of adenohypophysis or a damaged state of adenohypophysis.

21. A therapeutic composition comprising a cell aggregate or the cell sheet produced by the method according to any one of claims 9 to 13 as an active ingredient.
